# EUROPEAN PATENT SPECIFICATION

(11) **EP 1 451 571 B1**
(45) Date of publication and mention of the grant of the patent: **21.08.2013**
(21) Application number: 02783500.8
(22) Date of filing: 05.11.2002
(51) Int. Cl.: G01N 33/00, A01K 67/00, A01K 67/027, A01N 43/04, A61K 31/70, A01N 63/00, A01N 65/00, C12N 5/00, C12N 5/02

(54) **DEVICES FOR DOSING AND ADMINISTRATION OF THERAPEUTIC MICRO-ORGANS IN LIVING SUBJECTS**
VORRICHTUNGEN FÜR DOSIERUNG UND VERABREICHUNG THERAPEUTISCHER MIKROORGANE IN LEBENDEN INDIVIDUEN
DISPOSITIFS DE DOSAGE ET ADMINISTRATION DE MICRO-ORGANES THÉRAPEUTIQUES CHEZ UN SUJET VIVANT

(30) Priority: 05.11.2001 US 330959 P; 08.07.2002 US 393745 P; 08.07.2002 US 393746 P
(43) Date of publication of application: 01.09.2004
(73) Proprietor: Medgenics, Inc., 555California Street Suite 365 San Francisco, CA 94104 (US)
(72) Inventor: PEARLMAN, Andrew, L., 20164 D.N. Misgav (IL); BELLOMO, Stephen, F., 30900 Zichron Yaakov (IL); LIPPIN, Itzhak, 42920 Moshav Beit Yitzhak (IL); ALMON, Einat, 23840 Timrat (IL); PIVA, Guillermo, A., San Ramon,CA 94583 (US); GARFINKEL, Leonard, I., "deceased" (US); ROSENBERG, Lior, 84965 Omer (IL); SHAVITT, Menachem, D., 20142 D.N. Misgav (IL); SHANI, Noam, Zikron Yaakov (IL); BUKHMAN, Mordechay, Netanya 4201804 (IL); SHER, Niv, Haifa 26501 (IL)
(74) Representative: Pearl Cohen Zedek Latzer UK LLP
(86) International application number: PCT/IL2002/000877
(87) International publication number: WO 2003/040686

(56) References cited:
- WO-A-01/00859
- WO-A-03/039382
- WO-A1-91/02049
- WO-A1-99/21584
- US-A- 1 516 071
- US-A- 4 773 418
- US-A- 5 843 182
- US-A- 5 888 720
- US-B1- 6 197 575
- WRIGHT J.R. ET AL.: 'Cell therapy for diabetes using piscine islet tissue' CELL TRANSPLANTATION vol. 10, 2001, pages 125 - 143, XP002966122
- DOMASHEHNKO A. ET AL.: 'Efficient delivery of transgenes to human hair follicle progenitor cells using topical lipoplex' NATURE vol. 18, 15 April 2000, pages 420 - 423, XP002966123

## Description

### FIELD OF THE INVENTION

The invention relates to the field of tissue based micro-organs such as therapeutic tissue based micro-organs and, in particular, a micro-organ processing system.

### BACKGROUND OF THE INVENTION

Various methods for delivering therapeutic agents are known. For example, therapeutic agents can be delivered orally, transdermally, by inhalation, by injection and by depot with slow release. In each of these cases the method of delivery is limited by the body processes that the agent is subjected to, by the requirement for frequent administration, and limitations on the size of molecules that can be utilized. For some of the methods, the amount of therapeutic agent varies between administrations.

This document describes methods and apparatus for the production and use of therapeutic micro-organs, referred to herein as TMOs for the production and/or administration of therapeutic agents.

In general, some methods and uses of micro-organs and therapeutic micro-organs are described in US Patent 5,888, 720, PCT application PCT/IL01/00979, EP application 01 204 125.7 and US Patent application 09/589,736. These references also include reviews of the prior art, which is not repeated here. They also include information on possible uses of TMOs and the types of proteins that can potentially be generated.

US Patent 4,773,418 to Hettich describes a tool for the preparation of a skin transplant. The tool cuts or punches segments of a patient's original skin, and cuts or punches holes in foreign skin, i.e. cadaver skin or parts of an animal skin or skin substitute preparations on a synthetic base, for the purpose of making openings in the foreign skin, with attachments used to transfer pieces of the cut original skin into such openings.

US patents 5,888, 720 and 6,372, 482 to Mitrani and unpublished patent application 09/589,736, PCT/IL01/00979 and EP 01 204 125.7, provide some information regarding the preparation and maintenance of micro-organs, the preparation and maintenance of genetically modified micro-organs, information on nutrients and gasses in the maintenance and information on genetic modification possibilities

### SUMMARY OF THE INVENTION

Apparatus for the production of micro-organs and therapeutic micro-organs are described herein.

The invention provides a micro-organ processing system, comprising:
a plurality of operational modules, each the module performing all or part of a process of producing one or more micro-organs from a viable tissue explant, wherein the micro-organ is cut from the explant and is a viable tissue section having intact micro-architecture;
wherein one of the modules is a harvester module comprising a means for harvesting the tissue explant from a tissue, and
wherein at least one of the modules comprises an inlet for nutrients and an outlet for waste in order that the explant and/or the one or more micro-organs can be maintained; and
means for transferring the tissue explant or the one or more micro-organs from one module to a next module in micro-organ processing system, wherein the transferring is via ports in the modules and the means is carried out under sterile conditions.

The at least one module comprising an inlet for nutrients and an outlet for waste may be fitted with an inlet for supplying a transduction agent, such that the micro-organ can be genetically altered therein.

The at least one module comprising an inlet for nutrients and an outlet for waste may be fitted with a sampling outlet for sampling the surrounding fluid therein.

The plurality of modules may be supplied with matching ports and connecting mechanisms such that material can be transported between the modules without exposure to an outside environment.

The plurality of modules may carry out the process of producing one or more micro-organs under sterile conditions starting from the introduction of the tissue explant.

One of the plurality of modules in the micro-organ processing system may be a micro-organ module comprising a means for cutting the tissue explant into the one or more micro-organs.

The invention also provides a micro-organ processing station for the maintenance and optional genetic alteration of micro-organs, comprising:
the micro-organ processing system described above;
at least one port for docking a module or a plurality of linked modules;
a fluidics control system operative to control the flow of fluids to and from at least one of the modules; and
a power control system operative to supply motive power to elements within at least one of the modules.

The micro-organ processing station may also comprise a vacuum control system operative to supply a controlled vacuum to at least one of the modules, wherein the vacuum is for holding a tissue explant and/or the one or more micro-organs within at least one of the modules.

The fluidics control system may be operative to control the introduction of at least one material that causes the genetic alteration of the one or more micro-organs in one of the modules.

The micro-organ processing station may also comprise a sampling mechanism for sampling fluids from at least one of the plurality of modules.

The micro-organ processing station may further comprise an analyzer for analyzing the fluids for one or more of the process parameters including glucose, lactate, dissolved oxygen, dissolved carbon dioxide, ammonia, glutamine, pH, contaminants, or a secreted therapeutic agent. Optionally, the analyzer may analyze the fluids for the therapeutic agent secreted by the one or more micro-organs. In an embodiment, the micro-organ processing station may also comprise a controller for monitoring the amount of the therapeutic agent and indicating when the one or more micro-organs is suitable for implantation.

The micro-organ processing station may additionally comprise a means for enhancing the genetic alteration of the one or more micro-organs. Optionally, this means for enhancing may comprise mechanical agitation, electroporation or electromagnetic field creation, or any combination thereof.

### DEFINITIONS AS USED HEREIN

The term "explant" as used herein refers to a removed section of living tissue from one or more organs of a subject.

The term "micro-organ" as used herein refers to a tissue structure derived from an explant that has been prepared in a manner conducive for cell viability and function, while maintaining at least some *in vivo* interactions. Micro-organs are comprised of two or more adjacent layers of tissue, retain the micro-architecture of the organ or organs from which they were derived, and enable passive diffusion of adequate nutrients and gases to its cells and diffusion of cellular waste out of said cells so as to minimize cellular toxicity and concomitant death due to insufficient nutrition and accumulation of waste.

The term "donor" as used herein refers to a subject, from which the explant is removed that is used to form one or more micro-organs.

The term "therapeutic micro-organ (TMO)" as used herein refers to a micro-organ that has been genetically altered to produce a therapeutic agent, such as a protein. The therapeutic agent may or may not be a naturally occurring body substance.

The term "implantation" as used herein refers to introduction of one or more micro-organs or TMOs into a recipient, wherein said micro-organs or TMOs may be derived from tissues of the recipient or from tissues of another individual or animal. The micro-organs or TMOs can be implanted by grafting into the recipient's skin, by subcutaneous implantation, or by placement at other desired sites within the recipient.

The term "recipient" as used herein refers to a subject, into which is implanted one or more micro-organs or TMOs.

For clarity and completeness of presentation all aspects of the production and utilization of TMOs are described in this document and are described from the start of the processes to their end. It should be understood that each of the aspects described herein can be used with other methodologies and/or equipment for the carrying out of other aspects and can be used for other purposes, some of which are described herein. The present disclosure includes portions devoted to the preparation and maintenance of micro-organs for transformation into TMOs. It should be understood that the micro-organs, produced according to these disclosures can be used for purposes other than for transformation into TMOs.

In general, production of TMOs includes (1) obtaining a sample of a tissue, from a patient or animal to be treated or from another person or animal of the same or a different type, (2) producing a viable micro-organ or structure of micro-organs from the tissue (3) genetically altering the micro-organ, and (4) preferably, verifying the production of a desired agent (for example proteins) by the altered micro-organ (TMO). Utilization of the TMO includes production, within a patient's or animal's own body, of therapeutic material, such as proteins, for treatment. For example, the TMO can be implanted into or grafted onto the skin of the subject to produce the agent in vivo. In the case of tissue from another subject, the implant is optionally protected from reaction by the recipients immune system, for example, by housing the TMO in an immunoprotective capsule or sheath. For example, a membrane can be positioned to surround the TMO, either by placing the TMO in a capsule prior to implant or otherwise. The membrane should have a pore size that is large enough to allow for the passage of nutrients waste and the therapeutic agent, but is small enough so that it does not pass cells of the immune system.

One broad aspect of some embodiments of the invention is concerned with apparatus for harvesting a sample of tissue, appropriate for making micro-organs. In an exemplary embodiment, skin tissue is used as the basis for the TMO. Alternatively, the tissue can be lung, intestine, muscle or liver tissue. Potentially, any tissue can be used. Various tissue types, such as, for example skin, lung, liver, have been shown as being suitable for producing micro-organs. The tissue to be harvested can be removed from the body by any means of removing tissue, known in the art, such as biopsy procedures. Preferably, the harvesting procedure keeps intact the micro-architecture of the tissue from which it is removed.

In an exemplary embodiment, for example when skin is the tissue being harvested, the tissue sample is harvested by lifting the surface of the tissue and cutting a section of the skin to a specified depth. The section is thick enough to include all of the desired layers of the skin. Optionally, the desired layers include the entire epidermis and at least some portion of the underlying dermis (up to and including the full thickness of the skin) and corresponds in thickness from 0.3 to 3 mm, depending on the location of the skin from which the sample is taken. When a skin structure is used that includes both epidermis and some dermis (including all the cellular layers, matrix and stromal architecture of the dermis which compose it), and processing it into micro-organs, the viability of the harvested tissue can be maintained for long periods both *in vitro* and *in vivo,* following implantation. As used herein, the verbs "cut" and "slice" are used to denote separation of one portion of tissue from another using a sharp blade or blade-like object

After harvesting a suitable structure must be prepared, from the harvested sample, to be viable *in vitro* and preferably in vivo upon re-implantation. This sample preferably includes all the living layers and is thin enough so that nutrients from a medium in which the sample is kept can diffuse to all portions of the sample and waste products can diffuse to the medium for optional removal therefrom (or when the medium is refreshed). The distance from an external surface to each cell is preferably between 100 and 400 micrometers, although lesser or somewhat greater distances can also be viable. In fact distances as large as 500, 600 or even 1000 microns can be used successfully under certain circumstances. Of course, the slices themselves are twice as thick as the maximum distance.

The prior art methods described in the background hereof, are limited since they do not provide a means for stabilizing the tissue during the cutting process. Therefore, tissue slices obtained by these methods are generally not uniform in width and shape. In addition, the length of the micro-organ is limited and only simple parallelepiped shaped pieces can be formed, which may make processing and utilization of the micro-organs more difficult. Furthermore, the epithelial layer of skin is tough and the sharp edge tends to deform the sample while it is being sliced. In addition, skin tends to stick to any surface that it contacts, further complicating the cutting process.

An aspect of the disclosure is concerned with preparing micro-organs from tissue samples. In accordance with an exemplary embodiment the harvested skin sample is cut into micro-organs using a plurality of cutting blades in a single assembly driven against the skin sample on a support base using a stamping operation, rather than utilizing a simple cutting operation. In one embodiment, the blades are arranged in two sets. The blades of each set are interlaced with the blades of the other set, but are slightly misaligned along the length of the blades. When a sample of tissue is stamped with this cutter, alternate ends of the sample remain attached as in an accordion. When the sample is drawn out, a long sample of substantially uniform width and thickness (depth into the skin) is produced. As described herein, this sample is relatively easy to handle, has a relatively large volume of tissue and can be cut into any suitable length when ready for use. Since it is uniform, the production capability of therapeutic material of each section is substantially the same and determination of the length of a sample needed, for example for implantation in a subject, can be made, based on the production of therapeutic material by the entire sample, *in vitro.* This general process, although described above for producing linear structures, can be modified for producing mesh and other useful micro-organ structures, as well.

After the sample has been formed into a suitable micro-organ structure by the above means or by any other means, the micro-organ is optionally genetically altered. Any methodology known in the art can be used for genetically altering the tissue. One exemplary method is to insert a gene into the cells of the tissue with a recombinant viral vector. Any one of a number of different vectors can be used, such as viral vectors, plasmid vectors, linear DNA, etc., as known in the art, to introduce an exogenous nucleic acid fragment encoding for a therapeutic agent into target cells and/or tissue. These vectors can be inserted, for example, using any of infection, transduction, transfection, calcium-phosphate mediated transfection, DEAE-dextran mediated transfection, electroporation, liposome-mediated transfection, biolistic gene delivery, liposomal gene delivery using fusogenic and anionic liposomes (which are an alternative to the use of cationic liposomes), direct injection, receptor-mediated uptake, magnetoporation and others as known in the art. This gene insertion is accomplished by introducing the vector into the vicinity of the micro-organ so that the vector can react with the cells of the micro-organ. Once the exogenous nucleic acid fragment has been incorporated into the cells the production rate of the therapeutic agent encoded by the nucleic acid fragment can be quantified.

As indicated above, the micro-organ is in contact with a nutrient solution during the process. Thus, a therapeutic agent generated by the micro-organ is secreted into the solution where its concentration can be measured.

The micro-organ, genetically altered or not, can be utilized in several ways. One is to implant it (or part of the total amount that has been generated) into a subject. In an important exemplary embodiment of the invention, the TMO is implanted in the same subject from whom it was taken. For example, genetically altered skin may be implanted under or grafted onto the skin of the subject. Tests in animals have shown that such an implant will continue to produce the therapeutic agent for a considerable amount of time, in vivo.

Alternatively, the TMO can be kept in vitro and the therapeutic agent whether left in the supernatant medium surrounding the TMO or isolated from it can be injected or applied to the same or a different subject.

Alternatively or additionally, the micro-organ or TMO can be cryogenically preserved by methods known in the art, such as for example, gradual freezing (0°C, -20°C, -80°C, -196°C) in DMEM containing 10% DMSO, immediately after being formed from the tissue sample or after genetic alteration.

The amounts of TMO implanted may be determined from one or more of:
Corresponding amounts of the same therapeutic protein routinely administered to such subjects based on regulatory guidelines, specific clinical protocols or population statistics for similar subjects.
Corresponding amounts of the same therapeutic protein specifically to that same subject in the case that he/she has received it via injections or other routes previously.
Subject data such as weight, age, physical condition, clinical status.
Pharmacokinetic data from previous TMO administration to other similar subjects.
Response to previous TMO administration to that subject.

In accordance with the invention, a closed modular apparatus may be used to carry, support and alter the micro-organ/TMO from its harvesting until implantation. Ideally, the entire process is carried out using closed, optionally sterile, modules, with transfer of the micro-organ/TMO taking place between modules under sterile, controlled circumstances, without manual handling of the micro-organ/TMO.

In an embodiment of the invention, the modules have matching ports so that there can be an easy transfer of micro-organ/TMO between the modules and so that the modules can cooperate to carry out the processes.

In accordance with an aspect of some embodiments of the invention, the modular apparatus is loaded into one or more of a series of docking stations, in which the processes are carried out and/or in which the micro-organ/TMO is maintained. This process is optionally computer controlled according to a protocol.

Optionally, only a portion of the TMO generated is used in a given treatment session. The remaining TMO portion is returned to maintenance (or is stored cryogenically or otherwise), for later use.

Optionally, a large amount of micro-organ is processed into a TMO together. This allows for easier and more exact determination of the secretion of the TMO and for determination of dosage.

Thus, the apparatus of the invention may be used in a method of harvesting a tissue sample from a subject, comprising:
attaching an outer surface of a portion of the tissue sample to a substantially flat surface area;
lifting the surface area, together with the portion of the tissue; and
slicing the tissue at a given distance below the surface area of said portion to separate the tissue portion from the subject. Optionally, said attaching is provided by vacuum suction. Alternatively or additionally, said attaching comprises providing an adhesive surface to said substantially flat area.

The lifting may comprise placing a support element between an extension of the flat surface area such that the surface of the portion is raised by substantially said given distance above the level of surrounding tissue surface. Optionally, slicing comprises slicing the tissue at a level substantially equal to a surface of the support element distal from said tissue surface.

In an exemplary embodiment of the invention, The tissue may be skin and the tissue surface may be the outer surface of the skin. Optionally, the given distance is between 0.3 and 3 mm. Optionally, the distance is between 0.5 and 1.5 mm.

The dimensions of the tissue that is harvested may be between 3 and 150 mm. Optionally, the minimum dimension is between 5 and 20 mm. Alternatively or additionally, the maximum dimension is between 20 and 60 mm.

The curting may be carried out using a dermotome comprising:
a tissue carrier adapted to adhere a tissue surface to a surface thereof;
a lifter adapted to lift a portion of said tissue surface; and
a cutting blade configured to cut the tissue, substantially parallel to said surface, at a controlled distance below said surface. Optionally, the carrier surface is formed with holes and further comprising a source of vacuum that provides a vacuum at said holes, thereby to provide said adaptation for adhesion. Alternatively or additionally, the carrier surface is an adhesive surface, thereby to provide said adaptation for adhesion.

Optionnally, said cutting blade is moved from side to side while advancing, to provide a slicing action. Alternatively or additionally, the cutting blade is at a controlled distance from the carrier surface during said cutting. Alternatively or additionally, the cutting blade is at a controlled distance from the skin surface during said cutting. Optionally, the dermotome includes a guide that sets said blade at said controlled distance.

The micro-organ structure may comprise at least two micro-organ portions formed from a tissue, in which said at least two micro-organs are linked one to the other by means of a junction formed from said tissue of which the micro-organs were formed therefrom. Optionally, at least three micro-organs are attached to one another via the same junction formed from the same tissue as said micro-organs. In an exemplary embodiment of the invention, a micro-organ mesh structure formed of junctions of micro-organs as described above is provided, in which a multiplicity of said junctions are interconnected by micro-organs, where at least one such micro-organ is attached to one of said junctions at one end of the micro-organ and is also attached to another junction at the other end of said micro-organ.

The micri-organ may comprise a segmented linear micro-organ structure, having interlinking junctions, in which at least some of the interlinked junctions have two linear micro-organs connected to it. Alternatively, the segmented linear micro-organ structure, may have interlinking junctions, in which at least some of said interlinked junctions have more than two linear micro-organs connected to it. Optionally, each interlinked junction has four linear mirco-organ structures connected to it. Optionally, the linear micro-organ structures and the junctions form a mesh structure.

The linear micro-organs and the junctions may form a super-linear structure comprising a string of alternating micro-organs and junctions.

Optionally, the micro-organ is comprised of a plurality of tissue layers and wherein the junction comprises the same layers. Optionally, the junction is a micro-organ.

In a micro-organ structure, the micro-organ may be comprised of a plurality of tissue layers, wherein the junction comprises fewer layers than the micro-organ.

The tissue in the micro-organ may be a human skin tissue.

The system of the invention may be used in conjunction with a device for the preparation of micro-organs from a volume of tissue by cutting, comprising;
a) a cutting array comprising a plurality of adjacent cutting blades that are disposed in close proximity and parallel to one another along at least one segment of their respective lengths and maintained at approximately equidistant from one another along said segment, such that the cutting edges of said adjacent cutting blades are separated by a slice distance between about 200 micrometers to about 2000 micrometers along said segment;
b) a tissue sample carrier, adapted to hold a slice of tissue, such that when said tissue, held on said carrier is pressed against said cutting array, said tissue is sliced by said cutting blades. Optionally, the device comprises:
   a removal mask comprising at least one insert which fits between the parallel segments of the cutting blades without hindering the cutting action of the blades. Optionally, the at least one insert comprises a plurality of said inserts. Optionally, said plurality of inserts are linked together so that they can be inserted or removed from between the cutting blades together. Alternatively or additionally, the plurality of linear inserts are attached together at ends thereof.

The device may include means for applying pressure between said sample carrier and said cutting array.

Optionnaly the cutting blades all have the same length such that they cut a plurality of linear micro-organs from the tissue sample.

The cutting blades may have substantially the same length, but are longitudinally offset from each other such that they cut a plurality of linear micro-organs connected at alternate ends thereof to an adjoining linear micro-organ by a junction micro-organ structure.

The cutting array may comprises at least three pluralities of blades each arranged end to end in a linear array of blades spaced by a given spacing, wherein said linear arrays are arranged side by side, with the spaces of one array being situated between the spacings of adjoining arrays. Optionally, the given spacing is between 0.5 and 2 times the slice spacing.

In an exemplary arrangement, the cutting blades all have the same length and are not offset from each other, and wherein a cutting edge of the blades at alternate ends of adjoining blades is below the edge of the remaining extent of the blade, such that the blades cut a plurality of linear micro-organs from the tissue connected by a junction that is less than the full thickness of the tissue.

In an exemplary arrangements, the cutting blades all have the same length and are not offset from each other, and wherein the tissue holder is formed with depressions at positions corresponding to alternate ends of adjoining blades such that the blades cut a plurality of linear micro-organs from the tissue connected by a junction that is less than the full thickness of the tissue.

The cutting array may comprise at least three blades, said blades having, periodically spaced thereon, cutting edges below the surface of the cutting surface of the rest of the blade wherein said linear arrays are arranged side by side, with the lower cutting surfaces of one blade being situated between the lowered cutting surface of adjoining blades, such that a series of junctions having a thickness less than the thickness of the tissue samples is formed at the lowered cutting surfaces.

The cutting array comprises at least three blades, and wherein the tissue carrier is formed with a plurality of parallel arrays of periodically spaced depressions thereon, corresponding to positions of said cutting blades, with the depressions of one array being situated between the depressions of adjoining arrays, such that a series of junctions having a thickness less than the thickness of the tissue samples is formed at the depressions.

In an exemplary arragement, said blades are arranged as a series of concentric circles, spaced by said slice spacing, such that a plurality of micro-organs having a ring shape are produced from a tissue.

The blades, as described above, may have the form of a continuous spiral spaced by said slice spacing, such that a micro-organ in the form of a spiral is produced.

The tissue carrier may be adapted to hold said tissue by vacuum. Alternatively or additionally, said tissue carrier is adapted to hold said tissue by adhesion to a surface of the carrier.

it is appreciated that micro-organs or micro-organ structures may be produced from a tissue by
a) providing tissue of a suitable thickness from which to form the micro-organs;
b) providing a device as described above;
c) placing the tissue on the sample carrier of said device;
d) placing tissue on the carrier into intimate contact with the cutting blades of said device; and
e) pressing said tissue against said blades until at least part of said tissue has been cut through a thickness thereof, thereby creating at least one micro-organ or micro-organ structure.

Another method for producing accessible micro-organs or micro-organ structures from a tissue by cutting, comprises
a) providing tissue of a suitable thickness from which to form the micro-organs;
b) providing a device as described above, such that said inserts are placed between said blades;
c) placing the tissue on the tissue carrier of said device;
d) placing tissue on the carrier into intimate contact with the cutting blades of said device;
e) pressing said tissue against said blades until at least part of said tissue has been cut through a thickness thereof, thereby creating at least one micro-organ or micro-organ structure; and
f) removing the at least one micro-organ or micro-organ structure from between the cutting blades by removing the mask from between said cutting blades, thereby disposing the cut micro-organs on the surface of the removed mask.

Optionnally, the pressing comprises rolling a cylindrical drum from one end of the carrier to the other, cutting the tissue as it rolls.

A method of producing a micro-organ from a tissue sample, may comprise
providing a thin tissue sample having a thickness and an extent in directions perpendicular to the thickness; and
cutting the sample through the thickness thereof over at least part of the sample to produce a micro-organ having at least one dimension smaller than 2000 micrometers and at least one other dimension larger than a largest dimension of the extent. Optionally, the cutting comprises a stamping action. Alternatively or additionally, the thin tissue sample is a thin, substantially rectangular tissue sample and wherein the cutting is in the form of a series of substantially straight cuts substantially parallel to one end said cuttings having a similar length and being offset lengthwise from each other so as to leave a junction of said tissue between alternative strips tissue at alternating ends of the cuts. Optionally, the method includes unfolding the thus formed cut sample to produce an extended micro-organ comprising strips of tissue connected by said junctions.

The cutting may comprise cutting in a spiral shape. Optionally, the method includes unwinding the spiral to provide an extended elongated microorgan.

Optionally, the cutting comprises cutting the tissue with a series of concentric circular cuts.

There is also provided in accordance with an exemplary embodiment of the invention, a Another method of producing a micro-organ from a tissue sample, may comprise
providing a thin tissue sample having a thickness and an extent in directions perpendicular to the thickness; and
simultaneously cutting the sample through the thickness thereof with a plurality of cuts, said cuts being formed in rows in an elongate direction of the cuts, each row having a plurality of spaced cuts spaced by a pitch and separated by spaces, cuts in alternate rows being offset in the direction of the cuts, so that the spaces of a given row are situated adjacent to a cut portion of an adjacent row. Optionally, the offset is equal to approximately one-half the pitch and the cuts of each row are substantially centered at the spaces of adjacent rows. Alternatively or additionally, the distance between adjacent cuts is between 200 and 2000 micrometers. Optionally, the distance is between 500 and 1500 micrometers.

In an exemplary embodiment of the invention. The distance between adjacent cuts may be between one-fifth and five times the distance between adjacent rows. Alternatively or additionally, the distance between adjacent cuts is between one-half and twice the distance between adjacent rows.

The spacing may be approximately equal to the distance between adjacent rows.

The method may include stretching said cut tissue sample so that it forms a mesh.

The cutting may comprise a stamping action. The thin tissue sample may be a skin tissue including at least the basal layer of the epidermis and a portion of the dermis. Optionally, the tissue sample includes the entire epidermis. Alternatively or additionally, the tissue sample includes stratum corneum. Alternatively or additionally, the tissue sample includes a majority of the dermis. Alternatively or additionally, the sample includes substantially the entire dermis.

Typically, the thin tissue sample is between 0.3 and 3 mm thick. Optionally, the tissue sample is between 0.5 and 1.5 mm thick.

The distance between cuts may be between 200 and 2000 micrometers. Optionally, the distance between cuts is between 500 and 1500 micrometers.

The ratio of the length of the blades to the spacing between the blades may be between 1 : 1 and 100:1.

There is thus provided in accordance with an exemplary embodiment of the invention, a micro-organ processing system, comprising:
a plurality of operational modules, each said module performing all or part of a process of producing said micro-organ from a tissue sample; and
means for transferring the tissue sample or micro-organ from one module to a next module in the process, via ports in the modules, without removal of the tissue sample from the modules. Optionally, one of the modules is a tissue harvester module that is pressed against the tissue and harvests a surface slice of tissue of controlled thickness. Optionally, said harvester harvests a surface slice of tissue of controlled width and length. Alternatively or additionally, one of the modules is a micro-organ module, in which the tissue sample is cut into one or more micro-organs. Optionally, the tissue sample is held on a sample carrier and stamped onto a cutter, while still being held on said carrier, to form a micro-organ.

In an exemplary embodiment of the invention, one of the modules is a micro-organ module in which the tissue sample is cut into one or more micro-organs.

In an exemplary embodiment of the invention, the tissue is cut in a meandering cut, such that the thus formed micro-organ has an unexpanded accordion shape.

In an exemplary embodiment of the invention, the micro-organ is transferred to a further module while developing the micro-organ into a long, super linear shape, having a length longer than the tissue sample. Optionally, a leading edge of the micro-organ is transferred to the further module and wherein the developed micro-organ is transferred onto a holder therein. Optionally, the holder holds the micro-organ such that there is contact of the micro-organ with a surface only over a limited portion thereof. Optionally, the portion corresponds to less than 10% of the micro-organ. Optionally, the portion corresponds to less than 5% of the micro-organ.

In an exemplary embodiment of the invention, the further module is fitted with an inlet for nutrients and an outlet for waste such that the micro-organ can be maintained therein. Alternatively or additionally, the further module is fitted with an inlet for supplying a transduction agent, such that the micro-organ can be genetically altered therein. Alternatively or additionally, the further module is fitted with a sampling outlet for sampling the surrounding fluid therein. Alternatively or additionally, the further module is fitted with cutting apparatus adapted to cut the micro-organ herein into one or more smaller pieces.

In an exemplary embodiment of the invention, the system includes a transporting module having an arm adapted to enter a port in said further module and remove at least a selected portion of a micro-organ therefrom for transfer to said transporting module.

In an exemplary embodiment of the invention, the modules are supplied with matching ports and connect mechanisms such that material can be transported between them without exposure to an outside environment. Alternatively or additionally, said modules carry out the process under sterile conditions starting from the introduction of the tissue sample.

There is thus provided in accordance with an exemplary embodiment of the invention, a micro-organ processing station for the control of maintenance and optional genetic alteration of micro-organs, comprising:
at least one port for docking a module or a plurality of linked modules;
a fluidics control system operative to control the flow of one or more of fluids and waste to and from at least one of the modules; and
a power control system operative to supply motive power to elements within at least some of the modules. Optionally, the station includes a vacuum control system operative to supply a controlled vacuum to at least one of the modules, for holding materials within at least one of the modules. Optionally, the fluidics control system is operative to control the introduction of at least one material that causes the genetic alteration of a micro-organ in one of the modules.

In an exemplary embodiment of the invention, the station includes a sampling mechanism for sampling fluids from at least one module. Alternatively or additionally, the station includes an analyzer for analyzing the fluids for one or more of the process parameters including glucose, lactate, dissolved oxygen, dissolved carbon dioxide, ammonia, glutamine, pH, contaminants, or secreted therapeutic agent. Optionally, the analyzer analyzes the fluids for a therapeutic agent excreted by the micro-organ.

In an exemplary embodiment of the invention, the station includes a controller that monitors the amount of therapeutic agent and provides an indication when the micro-organ is suitable for implantation. Optionally, the station includes means for enhancing the genetic alteration of the micro-organ. Optionally, the means for enhancing includes mechanical or acoustic vibration.

In an exemplary embodiment of the invention, said power control system controls a cutting of a tissue into one or more micro-organs.

### BRIEF DESCRIPTION OF DRAWINGS

In the figures, identical and similar structures, elements or parts thereof that appear in more than one figure are generally labeled with the same or similar references in the figures in which they appear. Dimensions of components and features shown in the figures are chosen primarily for convenience and clarity of presentation and are not necessarily to scale. The attached figures are:

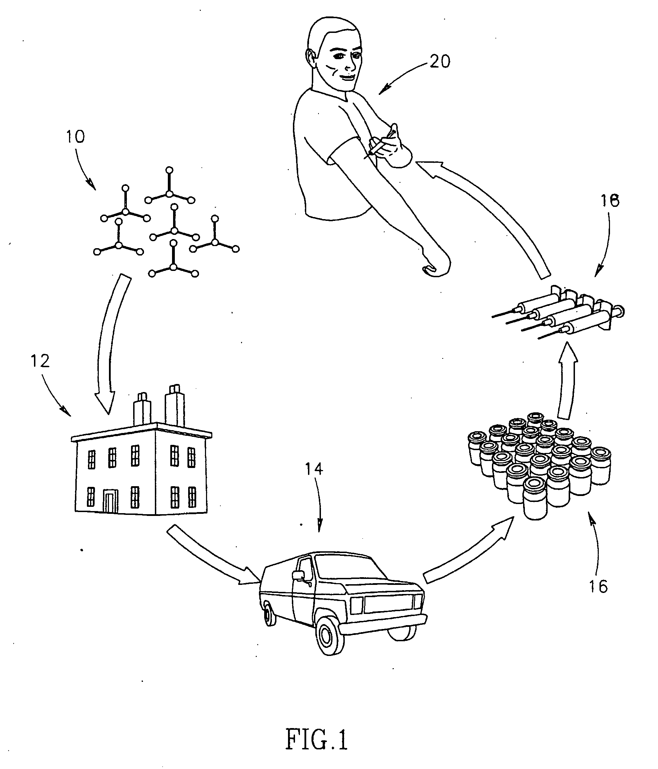

Fig. 1 is a schematic overview of an exemplary prior art "pharmaceutical" paradigm for the production and utilization of medications;
Fig. 2 is a schematic block diagram of an exemplary methodology for producing and utilizing genetically altered micro-organs (TMOs);
Figs. 3A and 3B illustrate a method of harvesting a skin sample from a subject;
Figs. 4A-4D show apparatus for the production of a micro-organ from a tissue sample, for example a skin tissue sample, such as that harvested using the method shown in Fig. 3;
Figs. 5A-5B show an exemplary blade structure for the production of a micro-organ from a tissue sample, for example a skin tissue sample, such as that harvested using the method shown in Fig. 3, and a resulting micro-organ;
Figs. 6A-6C show a mesh type micro-organ structure;
Fig. 7 shows a skin sample cut in a manner such that a mesh type micro-organ can be formed therefrom;
Fig. 8 schematically shows a tool for cutting the pattern of Fig. 7;
Figs. 9A and 9B schematically show the structure of fixtures for holding a super linear and a mesh patterned micro-organ, respectively during one or more of transportation, maintenance and genetic modification thereof;
Fig. 10 shows a simple Bio-reactor for processing micro-organs to produce TMOs;
Fig. 11 shows a tool being used to implant a TMO or micro-organ;
Figs. 12A-D illustrate successive steps in a first subcutaneous implantation procedure;
Figs. 13A-E illustrate successive steps in a second subcutaneous implantation procedure;
Fig. 14A shows a correlation analysis between in-vitro secretion of pre-implanted mIFNα-TMOs and the serum in-vivo levels following their implantation;
Figure 14B represents the pharmacokinetics of various injected recombinant therapeutic proteins in a subject, together with mIFNα produced and delivered by human skin TMO in SCID mice;
Fig. 15 shows the degree of variability of *in vitro* secretion levels from skin samples of different patients, processed at different times;
Fig. 16 shows elevated levels of erythropoietin in SCID mice after implantation;
Fig. 17 shows *in-vivo response* of erythropoietin in implanted mice as a function of a different numbers of implanted TMOs;
Figs. 18A and 18B show, respectively, elevated serum hEPO levels determined by an ELISA assay and reticulocyte count elevation after autologous TMO implantation in a miniature swine;
Figs. 19A-C, show hEPO protein in-vitro secretion detected after transduction;
Fig. 20 shows the main modules of a closed sterile micro-organ processing cassette, with the modules separated for ease of visualization;
Figs. 21 and 22 show the operation and details of a tissue harvester module;
Figs. 23-25 illustrate the formation of a micro-organ from a skin tissue sample;
Figs. 26-28 show some details of a TMO bio-processing module and the transfer of a micro-organ thereto;
Fig. 29 shows a processing station, having cassettes, each composed of a plurality of modules, mounted in it, in accordance with an embodiment of the invention;
Figs. 30-32 illustrate the cutting of a super-linear micro-organ into segments;
Figs. 33 schematically illustrates a transfer module;
Figs. 34-37 schematically illustrate removal of segments of micro-organs/TMOs from a bio-processing module and their transfer to a transfer module; and
Fig. 38 schematically illustrates transfer of a segment of micro-organ/TMO to an implantation holder.

### DESCRIPTION

### OVERVIEW OF THE SYSTEM

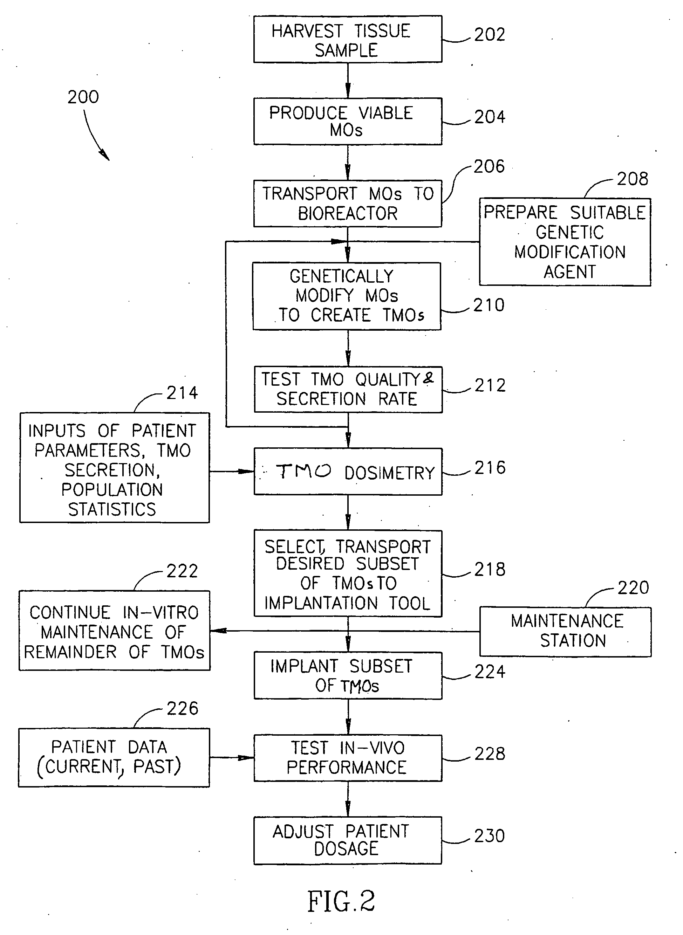

Fig. 2 shows an overview of a methodology 200 for producing and utilizing micro-organs and genetically altered micro-organs (TMO), in block diagram form. At 202 an explant of tissue is harvested from a subject, who may be the same subject to whom therapy will later be applied. In an embodiment the sample is a skin sample. Optionally, other tissues are harvested and used in a manner similar to that described below for skin samples. While the method described below is exemplary, other methods of harvesting tissue samples, such as coring, punching, etc., can be used. Furthermore, in general, any commercially available dermatome can be used. The harvested sample is optionally inspected to determine its condition and then transported to a micro-organ forming apparatus, optionally using the methodology described below. If desired, the tissue explant can be cryogenically stored for later use (i.e., introduction at the same stage of the process).

At 204 a viable micro-organ is produced from the explant. In order to be viable a micro-organ must have at least one dimension that is small enough so that nutrients can diffuse to all the cells of the micro-organ from a nutrient medium which contacts the micro-organ and so that waste products can diffuse out of the micro-organ and into the medium. This enables the micro-organ to be viable *in vitro* long enough for the further processing described below and for the optional further utilization of the micro-organ as a source for a therapeutic agent, such as a protein. The maximum distance from the outer surface of the micro-organ to any tissue that is to remain viable preferably should be less than about 1000 micrometers, although greater distances may also produce viable structures. The method of producing a micro-organ from a tissue sample, as described below, generally results in a micro-organ having an *in vitro* life of several months.

After the micro-organ is produced, it is optionally visually inspected to determine that it is properly formed and that it has the desired dimensions. Inspection can also be performed optically. It is then optionally mounted on a holder and transported (206) to an apparatus in which it can be genetically altered. A suitable genetic modification agent is prepared (208). Alternative methods of preparing the agent include creation of aliquots with a desired titer, using a predefined dilution buffer of viral particles, possible cryogenic storage and thawing of the viral aliquots under controlled temperature (0-4°C), and validating the activity of the viral vector. All of these processes are well known in the art. At this point the micro-organ can be stored cryogenically, for later introduction at the same place in the process. This can be performed using known protocols for gradual freezing of tissues and cells, using for example, DMEM medium containing 10%DMSO.

At 210 the micro-organ is genetically altered. As indicated in the summary, many methods of genetic alteration are known and can be used. As an example, the following description is based on using a viral vector to insert a gene into the cells of the micro-organ. This process is well known and will not be further described, except as to the particular methodology and apparatus for introducing the virus to the micro-organ.

At 212 the genetically altered micro-organ (TMO) is optionally tested for secretion rates of the therapeutic agent. There are various methods of determining the quantity of secretion, for example, ELISA, other immunoassays, spectral analysis, etc. In addition the quality of the secretion is optionally tested, for example for sterility and activity of the secreted protein. This may be performed periodically or continuously on line.

At this point the TMO can be cryogenically stored for later use.

At 214 and 216, the amount of TMO required for producing a desired theraputic effect is determined. As indicated below, the therapeutic dose requirements, can be estimated from measured secretion rates, patient parameters and population statistics on the estimated or known relationship between *in vitro* secretion and *in vivo* serum levels.

At 218 the selected portion of the TMO is transferred to an implantation tool. Exemplary implementation tools are described below. If needed, for allografts or xenografts the or for other reasons, the TMO is encapsulated. If the charged implementation tool (or the TMO) must be transported, it is optionally held (220) in a maintenance station, in which the temperature, humidity, etc. are held at a level that allows the TMO to survive during transport. The remaining TMO material is optionally maintained *in vitro* for future use. This can be at warm incubator conditions (37 °C), in conditions as described above or at cool incubator conditions (4 °C), which may prolog its viability *in vitro.*

At 224, a portion of the TMO (or a portion of the TMO produced by the previous acts) is implanted into the subject. There are a number of methods described herein for this implantation procedure. Other methods of doing so will occur to persons of skill in the art and are primarily dependent on the specific geometry of the micro-organ being used. Animal studies have shown that the micro-organs and TMOs remains viable *in vivo,* in the sense that the TMO continues to produce and secrete the therapeutic agent for a period of months after implantation. In animal studies, therapeutic amounts are produced for periods up to 120 days (or longer). While the tissue of the micro-organ or TMO appears to be integrated into the tissue of the subject into which it is implanted (especially if the tissue is implanted in a tissue of the same kind from which it was harvested), the cells comprising the micro-organ or the TMO continue to produce and secrete the therapeutic agent.

In an alternative approach, the therapeutic agent is harvested from the *in vitro* TMO and purified to remove nutrient and waste products. The purified agent is suitable to be injected or otherwise administered to a subject.

In either case, the *in vivo* performance of the TMO is optionally determined (228). Based on this evaluation for example, and/or on past patient data (226), patient dosage may then be adjusted (230) by increasing the amount of the implant or removing some of the implant, as described below. As the efficacy of the implant changes, additional TMO segments are implanted.

The following sections describe some of the above acts and variations thereon, in more detail.

### HARVESTING AN EXPLANT

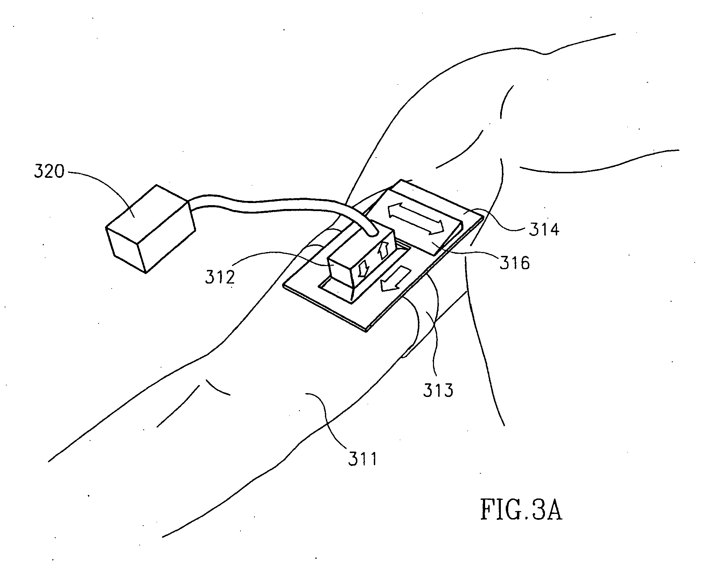

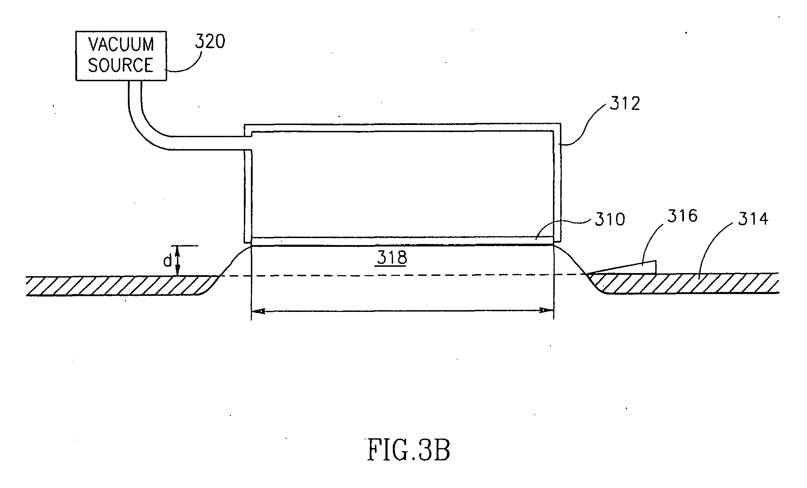

Figs. 3A and 3B schematically show, a method of harvesting a skin sample from a subject. A base plate 314 is placed against a donor site of a subject 311, from which a skin sample is to be harvested. The base plate is optionally pressed against the skin with a slight pressure, for example by a strap 313 around the arm or by other means. This base plate has a cutout window that defines the length and width of the tissue to be harvested and also serves to stabilize the skin around the donor site. A sample carrier 310 is located a known distance above the upper surface of the base plate (the depth of the tissue to be harvested). In an embodiment, sample carrier 310 is formed with small holes or slots and a vacuum head 312 is placed behind sample carrier 310 to draw the skin surface toward the sample carrier and hold it tightly against it at a predetermined height above the base plate, when a vacuum source 320 is activated. Various hole structures can be provided for different shapes of micro-organ structures shown below. The vacuum serves to stabilize the skin sample that will be harvested and to keep the harvested skin sample attached to the carrier after the tissue is removed from the person. Alternatively, an adhesive is placed on the sample carrier so that the skin will be attached to it. For example, a double sided adhesive is placed on the underside of sample carrier 310. If an adhesive is used, provision is made for allowing the sample carrier to touch the skin before being lifted away from the surrounding skin.

A thin, sharp blade 316 is drawn across the upper surface of the base plate to harvest a skin sample 318. The thickness of the sample on sample carrier 310, is determined by the distance of the upper surface of the base plate from the underside of sample carrier 310. The blade is optionally moved from side to side while it advances to facilitate the slicing of the sample. This side-to-side motion may be motorized or the entire motion may be by hand. In addition, the forward motion of the blade may be motorized. The vacuum head may remain attached to the sample carrier to prevent skin sample 318 from falling off sample carrier 310 during the commencement of the following process.

Typically, the skin sample is 6 mm wide by 35 mm long, by 1 mm thick. However, other length, width and thickness are useful. The lateral dimensions are not critical by any means. However, in producing consistent microorgans by the following process it is useful to have a standard size of explant.

### PRODUCTION AND MOUNTING OF MICRO-ORGAN

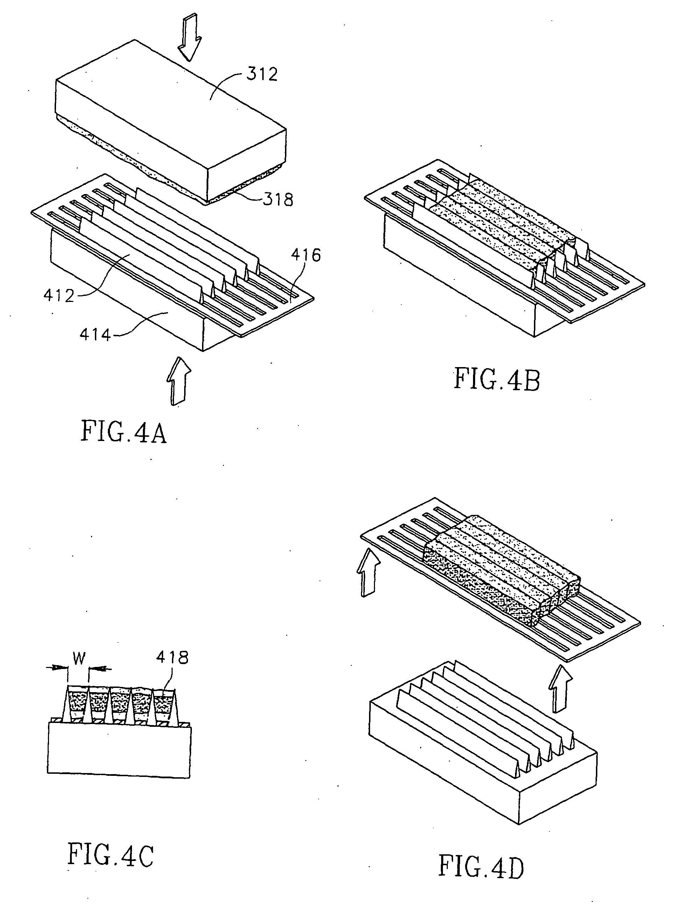

Figs. 4A and 4B show an apparatus 410 for the production of a micro-organ from a tissue sample, for example a skin tissue sample, such as that harvested using the method shown in Fig. 3.

In Fig. 4A, skin sample 318 mounted on sample carrier 310 (not visible) and held by vacuum head 312 is brought into contact with and pressed against a series of blades 412 mounted on a block 414. The blades are parallel and of the same length and are somewhat longer than the extent of the sample. A micro-organ mask 416 is set between blades 412, prior to tissue cutting. When the sample carrier is pressed against the blades with sufficient force, the tissue is sliced through and the skin sample (now micro-organs 418) is caught between the blades. If the micro-organs are held snugly between the blades, the carrier can be removed, along with the vacuum source of adhesive. Otherwise, the vacuum or adhesion is released. This is shown in Figs. 4B and 4C respectively, which are isometric and cross-sectional views of the micro-organs after slicing. The spacing between the blades, W, determines the width of the cut micro-organs. Micro-organ mask 416 is then lifted out of the blades (Fig. 4D) and the slices of micro-organ can then be removed for further processing. The pressing action described above may be aided by a press fixture or may be aided by rolling a rod over the top of the sample carrier.

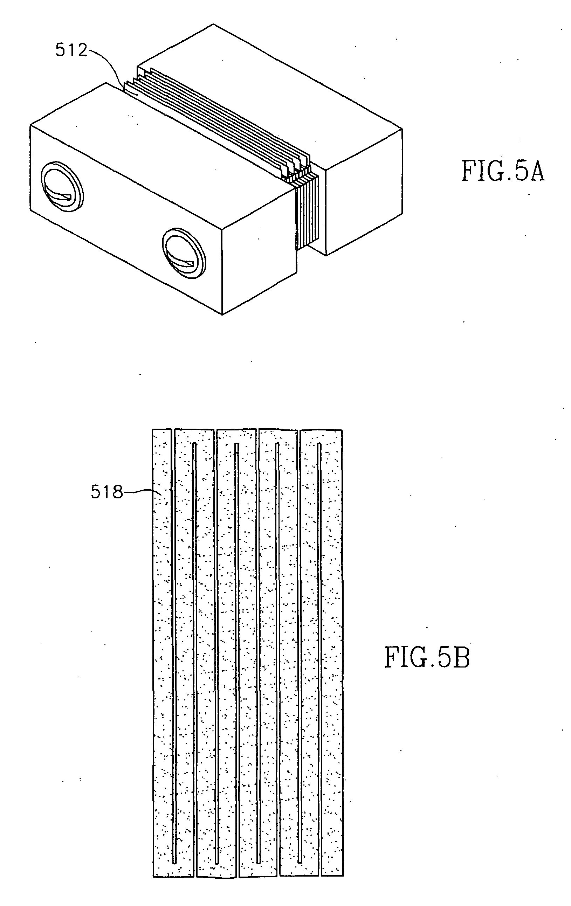

Fig. 5A show an isometric view of a structure for cutting a different shape micro-organ. In this structure blades 512 are divided into two groups that are axially offset from each other, as shown. A micro-organ mask similar to mask 416 is mounted between blades 512. When the skin sample is brought into contact with the blades and pressed against them, the skin sample is cut into a pattern as shown in Fig. 5B, where the cut skin sample (hereinafter the micro-organ) is designated 518. Micro-organ mask 416 is raised and micro-organ 518 is removed on micro-organ mask 416. As is evident in Fig. 5B, micro-organ 518, when cut is in a serpentine shape.

While in the method described above, the full thickness of the sample is preserved at the end "junctions" between linear structure, in general, only enough structure to hold the linear pieces together in the following processing is actually required. For example, for skin samples, it may be sufficient to leave only the epithelial layer. One approach is to have the blades aligned and of the same length. Rather indentations are formed in the tissue sample carrier (as in Fig. 3E), such that the blade will not cut through the entire depth of the tissue at these positions. This results in "junctions" that are only partial depth of the tissue

When the structure is developed (i.e., extended to its full length), the micro-organ is a very long structure, almost in the form of a parallelepiped. Due to its extreme length as compared to that of the original sample, this structure is sometimes termed herein as a "super-linear" structure. Other shapes are also possible. For example, if a spiral pattern is cut in the explant, the result is similar to that produced by the method described with respect to Figs. 5. Ring structure or rectangular thin walled structures can also be produced by stamping or cutting. Another variation of a large micro-organ structure is two adjacent linear micro-organs connected at both ends by junctions such that the structure can be opened to form a micro-organ tissue ring.

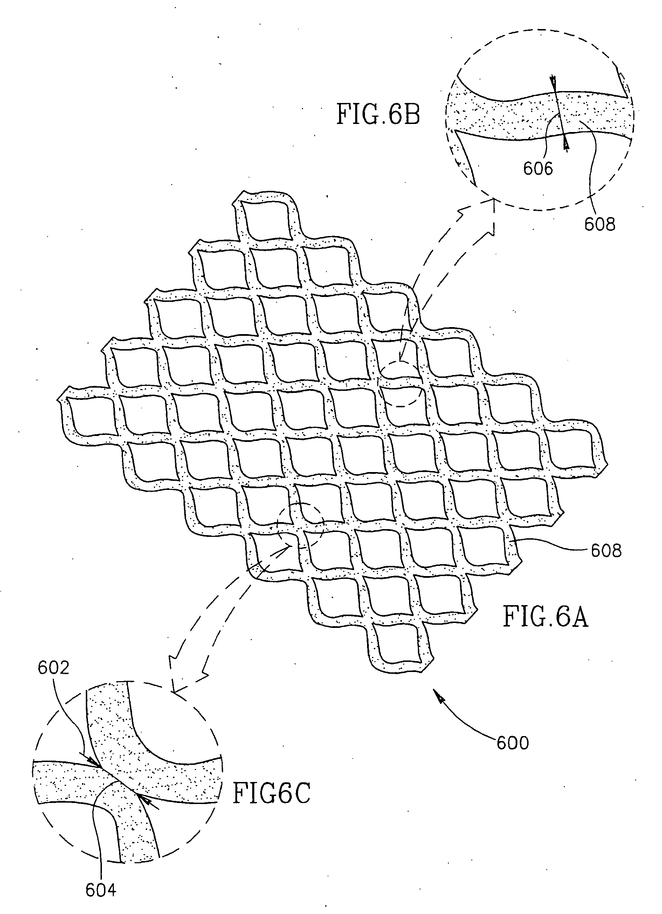

A schematic diagram of a mesh shaped micro-organ 600 is shown in Fig. 6A, with details of the mesh shown in Figs. 6B and 6C.

In viewing Fig. 6 it should be understood that the surface seen in the figures is either the outside of the skin layer (stratum corneum) or the opposite inner skin surface (lower dermis). Mesh structure 600 is designed such that each section of the mesh structure has a distance from a surface that allows it to receive nutrients and deliver waste products to a surrounding nutrient bath and, in addition, keeps the entire tissue sample intrinsically held together to simplify tissue handling and processing. Furthermore, the structure allows for the continuing identification of which side of the skin is which, so that the micro-organ or TMO can be implanted with a proper orientation, as described below.

As shown in Fig. 6B, if the width 602 of a junction 604 between two elements of the mesh is made equal to thickness 606 (Fig. 6C) of the arms 608 of the mesh (as shown), the area that is farther from nutrients than an innermost tissue of an arm 608 is very small. Also, it is only slightly more removed from sources of nutrients, etc. Making width 602 shorter further reduce both the area and distance. In some approaches, width is made equal to thickness 606. In others, it is greater or less than thickness 606. As can be seen in fig 6B, each segment of the mesh is substantially the same as a linear MICRO-ORGAN.

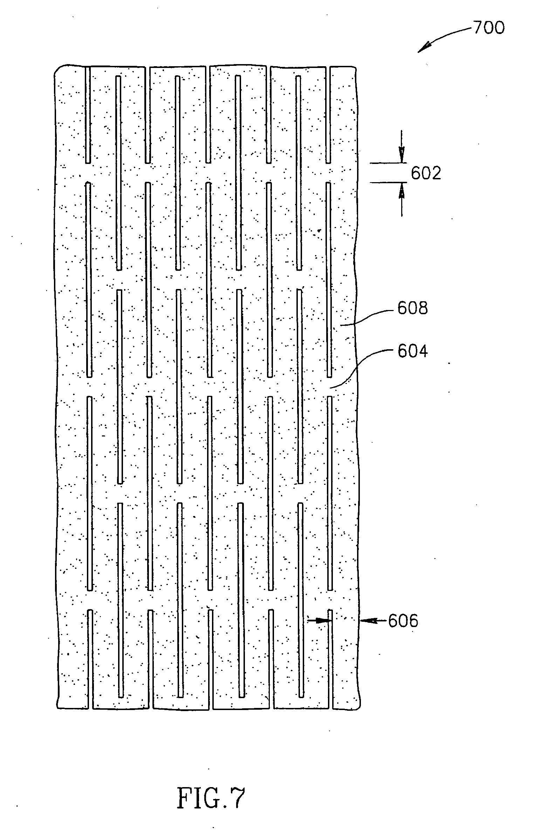

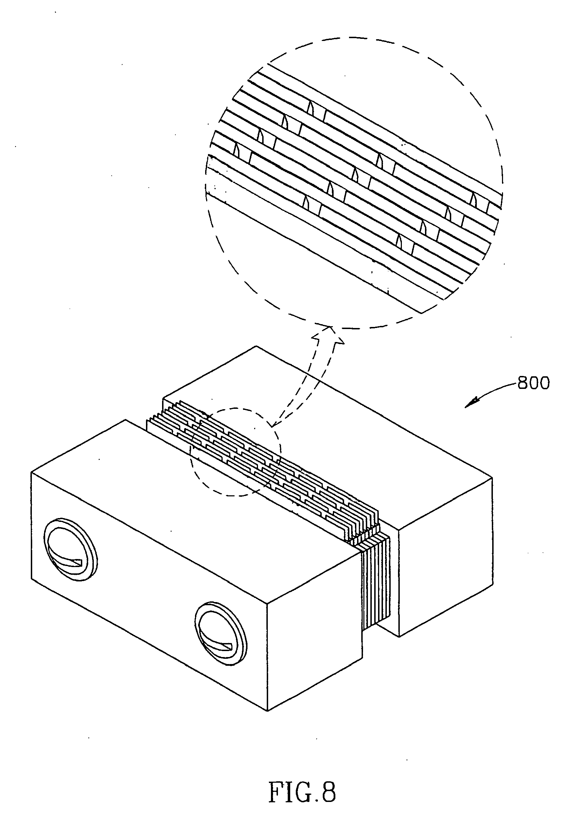

One way of producing a mesh, such as that shown in Figs. 6A-C is to press a pattern 700, such as that shown in Fig. 7, in a portion of tissue sample. It can be appreciated that the ratio of slit length to junction length can be a wide range of values ranging from a 1:1 ratio to a 1:100 ratio. This ratio will control the tightness of the mesh and the extent to which it can be expanded when stretched open. An exemplary blade cartridge arrangement 800 for press cutting (corresponding to apparatus 414 of Fig. 4A) is shown in Fig. 8. Alternatively, as mentioned above for the super-linear structure, unmodified equi-length blades can be used on a specially designed carrier which will create partial thickness junctions sufficient to hold the structure together as a mesh.

It should be understood that both the super-linear and mesh micro-organ structures as described above can be considered to be a construct of linear micro-organs connected together at junctions, which can be either micro-organs themselves or portions of non-micro-organ tissue.

After pressing a tissue to form a structure, as is Fig. 7, the tissue is laterally stretched to form the mesh shown in Fig. 6A. Reference numbers on Fig. 7, correspond to features referenced by the same numbers on Fig. 6A. The mesh can be stretched until the slits open into diamond shapes. Alternatively, the mesh is opened less than the maximum.

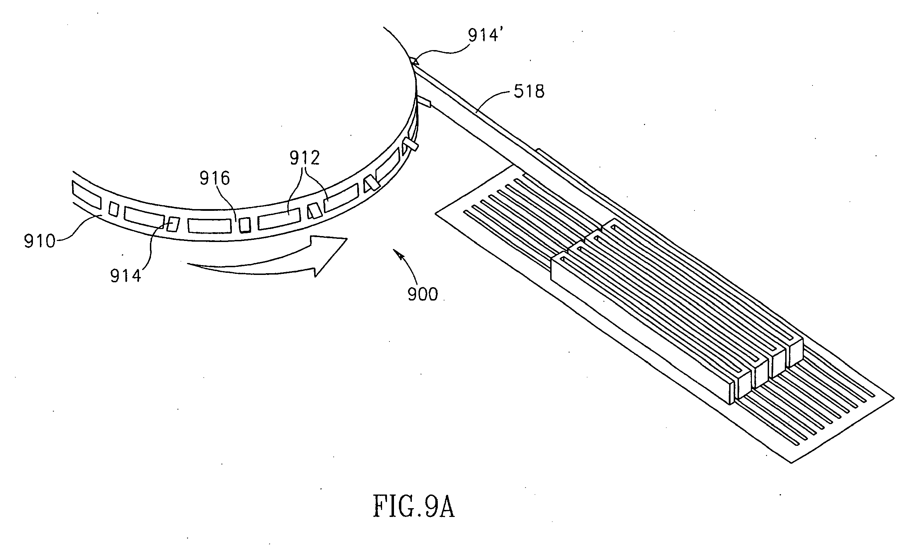

Fig. 9A shows an exemplary structure 900 used to hold the "super linear" micro-organ during processing. Another function of structure 900 is to facilitate the introduction and removal of the micro-organ into and out of a bio-reactor.

As shown in Fig. 9A, structure 900 comprises a substantially rectangular (but curved) body 910 formed with slots 912 having the same (or slightly greater) width than the tissue sample. The slots enable the free passage of fluids to both sides of the micro-organ.

Periodically along the length of body 910, clips 914 or other means for holding the micro-organ in place are formed. As micro-organ 518 is loaded onto body 910, the clips are closed to hold the micro-organ. The initial placement of the micro-organ into the clip may be effected by grabbing the end of the micro-organ with a vacuum pick-up tool, as known in the art. The first clip (indicated as 914') is shown as closed and holding the micro-organ. When the micro-organ is held on body 910 most of its surface area is exposed due to the freely exposed surface on one side of the micro-organ and the slots on the other.. This allows the micro-organ to be in good physical contact with its surround fluid or agents. This improves the viability of the micro-organ during the period it is *in vitro.* Alternatively, the clips (between the first and the last) are not closed on the micro-organ. Rather they are left open and the open clips keep the micro-organ from moving from side to side. Alternatively, the intermediate clips are replaced by elements, which are perpendicular to the surface of the holder which act to keep the micro-organ from slipping sideways.

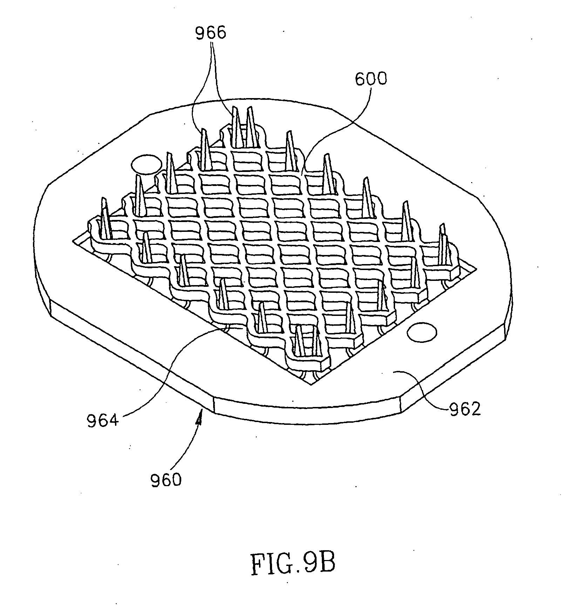

Fig. 9B shows a structure 960 used to hold the mesh micro-organ during processing. Another function of structure 960 is to facilitate the introduction and removal of the micro-organ into and out of a bio-reactor.

As shown in Fig. 9B, mesh type micro-organ 600 is mounted on a holder 962, having an aperture 964 in its central portion. A plurality of pins 966 is formed around the periphery of aperture 964. Mesh structure 600 is stretched, as described above, and held in the aperture by pins 966. Although pin or rod type holders are shown, other holders (such as clips), which hold the edges of the mesh, can be used. Furthermore, while a square aperture is shown, rectangular, circular or shapes having more than four sides can be used. While a completely opened mesh is shown, different dimensions and sizes of micro-organ can result in only partial opening of the mesh.

### MICRO-ORGAN BIO-REACTOR AND GENETIC ALTERATION

Once a micro-organ has been produced and mounted, it is ready for genetic alteration of the micro-organ to form a TMO.

Generally, genetic alteration comprises genetically engineering a selected gene or genes into cells that causes the cells to produce and optionally to secrete a desired therapeutic agent such as a protein. In an exemplary approach, at least parts of the process of sustaining the micro-organ during the genetic alteration and the genetic alteration itself are performed in a bio-reactor, as described above.

It is desirable for such a bio-reactor to have some or all of the following properties:
1) Allow for the provision of nutrients and gasses to the surfaces of the micro-organ so that they can diffuse into the micro-organ and the micro-organ can remain viable. Thus, significant areas and volumes of the micro-organ should not be blocked from coming into contact with a surrounding fluid.
2) Allow for the maintenance of the micro-organ at a desired temperature.
3) Allow for the maintenance of a desired pH and gas composition in the vicinity of the micro-organ.
4) Allow for the removal of waste products from the micro-organ and from the bio reactor.
5) Allow for a simple method of inserting the genetically modifying vector without substantial danger that the inserting vector will contaminate the surroundings.
6) Allow for the removal of excess unused vector.
7) Allow for measurement of the amount of therapeutic agent generated.
8) Allow for removal of substantially sterile therapeutic agent.
9) Allow for easy insertion of the micro-organ and removal of all or measured amounts of TMO.

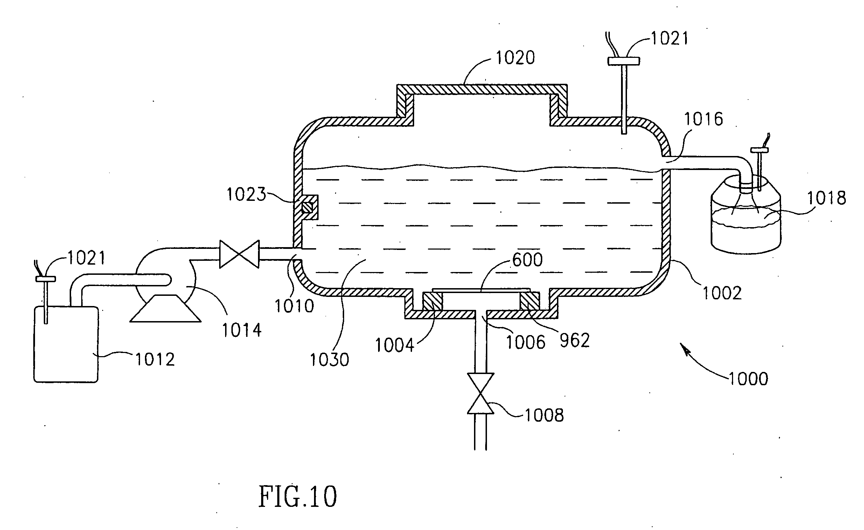

Fig. 10 shows a mostly cross-sectional schematic view of bio-reactor 1000. While bio-reactor 1000 does not have all of the desired qualities of an ultimate bio-reactor, it is illustrative of a simple useful model of a bio-reactor. While the structure shown is most suitable for use with a mesh type micro-organ held in a holder, as shown in Fig. 9, simple variants of the structure can be used for super-linear structures and for short linear structures of the prior art.

A container 1002, of plastic or some other non-reactive material is formed with a depression 1004 in its bottom. Depression 1004 is suitable for holding a micro-organ such as micro-organ 600 in holder 962 (Figs. 6A and 9B). A drain 1006, optionally in the lowermost portion of the container, is controlled by a valve 1008.

An input port 1010 is formed in the container. A nutrient solution, such as, for example, minimal DMEM including glutamine and antibiotics, optionally with dissolved gasses as necessary for the sustenance of the micro-organ is pumped into the container, from a nutrient reservoir 1012 by a pump 1014.

An overflow outlet 1016 is also formed in container 1002, such that any excess nutrient solution in container 1002 overflows into an overflow container 1018. A steady state fluid level is maintained, such that the average drainage flow-rate from the bio-reactor is equal to the inlet flow rate. Container 1002 is covered by a cover 1020, fitted with a suitable gasket system (not shown) so that it is gas tight and so that sterility is maintained. An optional air inlet 1021 (or outlet), filtered to preserve sterility in the container, is also provided for the container 1002, nutrient reservoir 1012 and overflow container 1018. The main reason for the air inlet is to preserve pressure equalization. However, a gas flow system is optionally provided above the nutrient level in container 1002 to control the concentration of oxygen and or other gasses. Optionally, gas can be dissolved in the nutrient liquid by bubbling gas through the nutrient in reservoir 1012 or container 1006.

In operation micro-organ 600 is inserted into container 1002, as indicated at 206 in Fig. 2. In one optional embodiment, the micro-organ holder can be physically attached to underside of cover 1020 by means of a rod or rods that correctly position the micro-organ inside container 1002 when the cover is closed. The container is partially filled with nutrient 1030 and held at a suitable temperature, close to body temperature. New nutrient is continuously pumped into the container and overflow nutrient (exiting via outlet 1016) carries with it a portion of the waste products produced by the micro-organ. Optionally, the nutrient is agitated mechanically or acoustically or by allowing the fluid flow mixing to mix the nutrient so that there is a steady flow of fresh nutrient to the micro-organ and so that waste products do not concentrate near the bio-organ.

Alternatively, an equal rate of nutrient is pumped into container 1002 and removed via drain 1006. Since drain 1006 is near the micro-organ and since the flow is always from the micro-organ to the drain, fresh nutrient is always delivered to the micro-organ and waste products are effectively removed. The inlet and outlet flow rates should be sufficient so that the necessary concentrations of nutrients and gasses is maintained, but not so great as to wash away the growth factors naturally produced by the micro-organs and necessary to maintain its viability when maintained in a minimal medium.

In either case, the nutrient material leaving container 1006 is optionally periodically or continuously checked to determine the level of glucose, lactate, ammonia, dissolved O₂, dissolved CO₂ and other nutrients such as amino acids. If the levels are outside a desired range, corrective action is taken.

After a specified latency period, typically of the order of 24 hours, which has been found to assist viral transduction of micro-organs. (or, optionally, immediately on insertion of the micro-organ in container 1002), nutrient 1030 is removed via drain 1006 and replaced by new nutrient containing a viral vector. Only enough nutrient solution need be provided to cover the micro-organ, but more may be used. Optionally, the viral vector is added by injection via a separate septum port 1023. Alternatively, it is delivered via port 1010 and a three-way connection in the line leading up to port 1010. Optionally, only a portion of the nutrient is removed and the remainder is used to supply nutrients during the gene insertion.

Optionally, the nutrient is not changed during the genetic modification process. After the process is completed, the virus containing nutrient material is drained from container 1002 and the container is filled and emptied one or more times to remove traces of the virus.

New nutrient solution is added and perfusion is continues for a specified time, typically on the order of days. This optionally allows for accurate characterization of the secretion levels and testing for sterility and the like.

Optionally, agitation of the micro-organ can be accomplished by any of the means listed above (shaking, rocking, rolling, fluid-flow mixing, acoustic agitation, etc.) during all the stages of processing, specifically during latency, transduction and maintainance post-transduction. The development of the secretion of therapeutic agent is then periodically checked, for example by measuring the concentration of therapeutic agent in the material removed via drain 1006 or outlet 1016. Optionally, corrective action can be taken, based on the secretion data, for example, an additional transudation can be carried out.

Furthermore, once the secretion levels of the desired agent from the micro-organ is known, this information can be used together with an appropriate pharmokinetic model and/or population statistics data to determine the number of micro-organs/TMOs needed to be returned to the subject in order to achieve the desired therapeutic effect in-vivo. The TMO is suitable to be implanted in or under the patient's skin or any other tissue so that it will remain viable, vascularize and maintain active physiological function, while producing and delivering the agent at desired levels for safe and effective therapy for extended periods. In some embodiments the micro-organs/TMOs may be implanted in the same subject from which the original tissue sample was taken (autologous). In another option, the micro-organs/TMOs can be implanted in a different subject (non-autologous). More information on implantation is given in a later section.

### IMPLANTATION OF THE MICRO-ORGANS/TMOS

Implantation of TMOs, has proven to be relatively simple and effective.

Before implantation a portion of the TMO must be removed from the Bio-reactor and prepared for implantation. For the examples of Figs. 9A and 9B holder 962 (or 900) on which the TMO is mounted is removed from the bio-reactor and the desired portion of the TMO is removed for implantation. The amount of material removed is optionally based on the measurements made on the secretion levels in the bio-reactor.

The full therapeutic potential of the TMO, is optimally achieved by implanting the TMO in subjects in need of therapeutic proteins. Procedures for implantation can have a significant effect on the efficacy and possible side effects of treatment using a TMO.

In order to maximize the efficacy of the TMO, the tissue should be introduced into the patient in such a way as to optimize the benefits of the therapeutic protein secreted by the TMO. For example, the TMOs can be implanted in regions where local protein delivery is required, or they can be implanted to provide (or optimize) systemic delivery. Optimally, the tissue being implanted should not be altered in any way or damaged during the procedure, since such damage could affect the therapeutic outcome of the treatment. In addition, it may be desirable, for the implantation procedure to be simple to perform, preferably not requiring the expertise of a plastic surgeon, dermatologist or other specialist. The procedure should also be performed quickly and with minimal pain for the patient being treated.

The number and size of TMOs that are implanted can control treatment dose. Whole or partial TMOs can be implanted or removed/neutralized to adjust the level of secretion in the patient. Multiple TMOs each generating a different therapeutic agent can also be implanted.

One method of grafting a linear TMO and two methods for subcutaneous implantation of a TMO are described below.

### Linear TMO Graft:

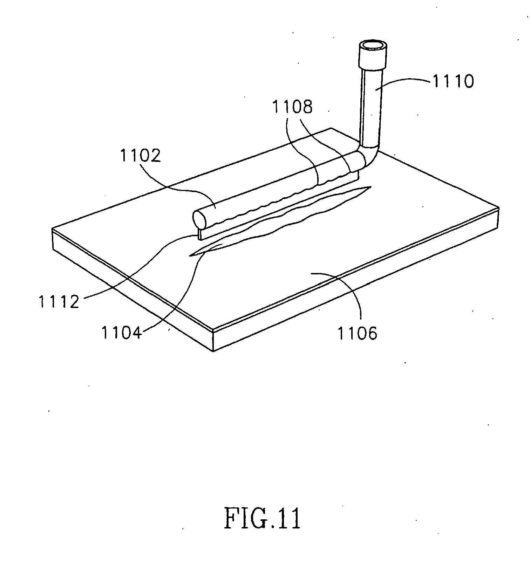

Fig. 11 shows a tool 1102 for implanting a length of TMO into a cut 1104 in a skin surface 1106. As shown in Fig 11 tool 1102 is formed with a plurality of holes 1108, connected via a tube 1110 to a vacuum source (not shown). The holes hold a length of TMO 1112 with its stratum corneal edge held by the vacuum. This vacuum pick-up tool is used to guide a TMO into slit 1104.

A linear TMO can be grafted onto a patient's skin by making an incision of an appropriate depth and length at the recipient site, placing the linear TMO in the incision and resealing the wound with the TMO in place. The grafted TMO becomes an integral part of the skin at the recipient site. For best results, the TMO orientation should be such that the stratum corneum, epidermis and dermal layers of the TMO line up with the corresponding layers of the surrounding skin tissue. Optionally, a scalpel used for making the cut is held in a structure that controls the depth of the cut. This scalpel tool used to make the slit should have a base plate with a window cutout that defines the length of the cut and provides a means for putting the surrounding skin under a slight tension prior to the incision. The scalpel tool is placed onto the base plate and allows for the scalpel tip to protrude approximately 1 mm below the bottom surface of the base plate such that the slit depth will be accurately controlled. Once the slit is made, the scalpel tool can be removed and replaced with a guide which is used to lower the vacuum pick up tool in the correct orientation such that the TMO on the tool is positioned correctly into the slit. Once in place, the tension in the surrounding tissue is relaxed such that the slit closes around the linear TMO graft and optionally a slight pressure can be applied to keep the wound closed. At this stage the vacuum can be disengaged and the vacuum tool along with the base plate can be removed.

Bandaging of the wound should ensure that the graft is not pushed out or exposed to the environment during the period of healing. The bandaging will optionally apply moderate pressure to the graft to hold it in place and assist in its integration. Protein produced by a grafted TMO is secreted into the skin tissue and enters the dermis and subcutaneous space. There is no concern over rejection of the graft because the TMO is an autologous skin sample.

### Subcutaneous Linear TMO Implantation:

A TMO implanted subcutaneously will remain in place (will not be pushed out) and is protected from minor trauma. Such implementation involves less external damage to the skin than a grafting procedure, and so is less painful and more aesthetic. The subcutaneous implantation procedure is more similar to an injection than to a surgical cutting procedure.

In a subcutaneous implantation procedure, a catheter is passed through a section of skin through the subcutaneous space such that the sharp end optionally comes out through the skin surface on the opposite side. In order to ensure a known length of passage of the catheter under the skin, the skin of the patient at the recipient site can be raised by some mechanical means, such as by a vacuum source or by lifting a stuck piece of double-sided tape, and the catheter can be passed through the base of this protrusion of skin. The length of the base can be defined by the size of the tool producing the vacuum or the size of the piece of double-sided tape used.

Once implanted subcutaneously, the TMO has access to the intracellular fluid in the subcutaneous space and all protein secreted passes into the subcutaneous space; this space is the same as the injection site of many bolus injections of therapeutic proteins.

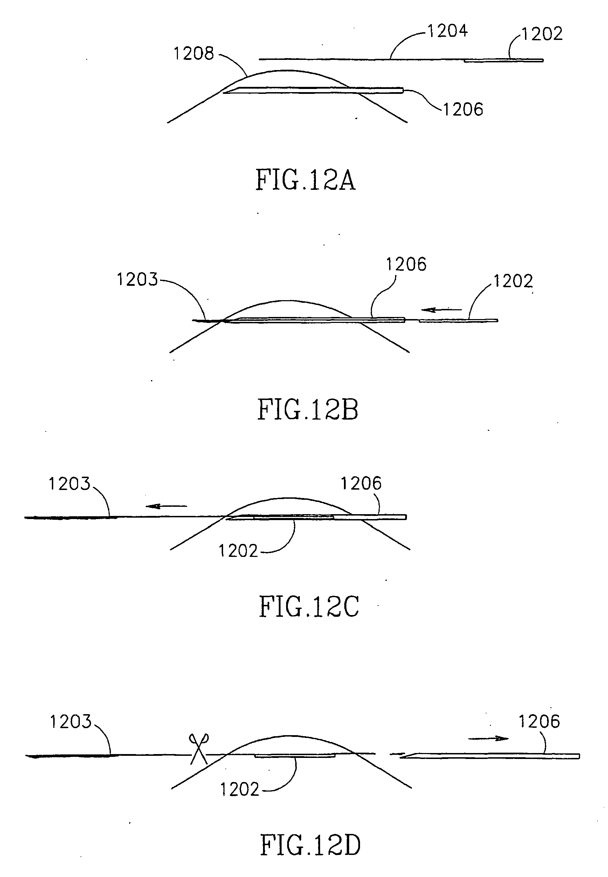

Figures 12A-D illustrate successive steps in a subcutaneous implantation procedure. In this procedure, in preparation for implantation a TMO 1202 is first attached to a surgical thread 1204 or other similar type of thread using, for example, titanium clips or other means of fastening. A catheter 1206 is inserted under the skin 1208 so that its end does not exit the other side (Fig. 12A). The thread can be stiff or flexible, absorbable or not, fabricated out of any biocompatible material and with a wide range of diameters. The thread has leading suture needle 1203 or other needle like object attached to its leading end, which is longer than the length of the catheter. The needle, with the attached thread and TMO clipped to it is introduced into the above-mentioned catheter (Fig. 12B), and penetrates the skin beyond the leading end of the catheter, thereafter being pulled through, until the TMO is positioned correctly in the subcutaneous space under the skin as described above (Fig. 12C). The practitioner holds the needle and/or thread while withdrawing the catheter, leaving the thread and the TMO in place (Fig. 12D). The thread can be trimmed at one end flush to the skin with a slight protrusion at only one end, or it can be made to protrude slightly at both ends.

The thread helps to mark the location of the TMO, thereby facilitating its identification and later removal if necessary in order to adjust or stop the protein therapy. More significantly, the thread provides a channel through which keratin produced by the skin of the TMO can pass out of the subcutaneous area. Keratin sloughed off from the stratum corneum of the TMO skin could accumulate in the region of the subcutaneous implant, causing the formation of inclusion cysts. The presence of the thread may cause the keratin to flow along the longitudinal axis of the thread and out of the body. The epidermis of the TMO will generate epithelial cells around the thread, so that a stable channel of keratin will form around the thread, in some situations.

In one variation on the above procedure, the catheter is placed in the subcutaneous space so that the sharp end comes out through the skin surface on the opposite side. A suture needle is then attached to the leading end of the surgical thread, which is in turn attached to the TMO. The rest of the procedure is as described above.

In another formulation, the thread can be made with hooked protrusions. This thread, without a needle, with the TMO attached to it, is loaded into the catheter prior to the placement of the catheter into the subcutaneous space. As above, the catheter is positioned, but does not protrude through the skin surface on the opposite side. When the catheter is positioned, it is immediately withdrawn and the hooks of the thread prevent the tread from being withdrawn together with the catheter.

In general, for subcutaneous procedures, the TMO can be unencapsulated or it can be encapsulated or enclosed in a membrane. The membrane should have a pore size that is large enough to allow for the passage of nutrients waste and the therapeutic agent, but is small enough so that it does not pass cells of the immune system.

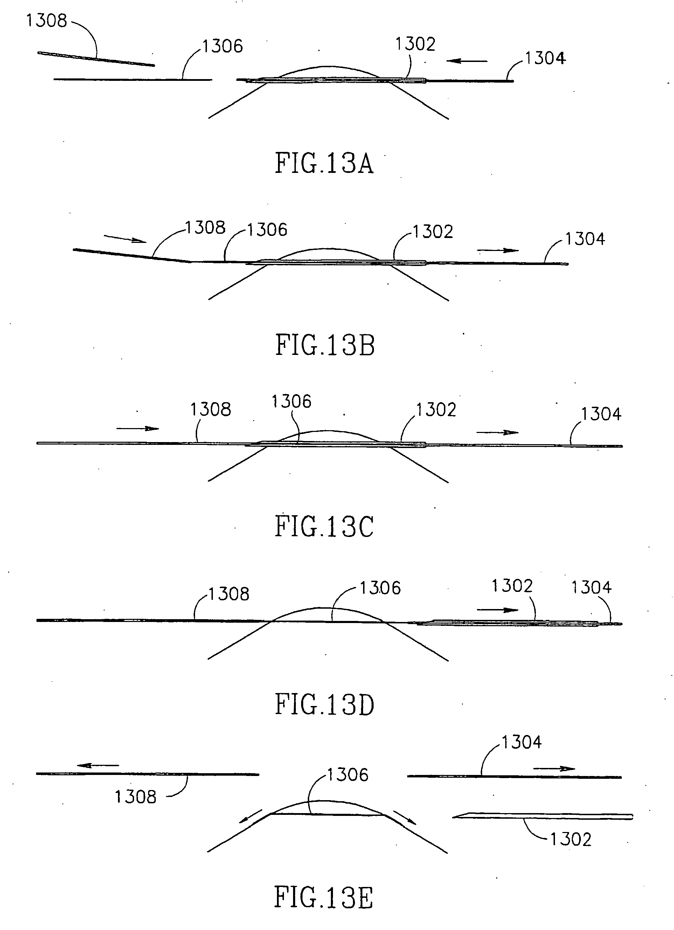

Figs. 13A-E illustrate successive steps in a second subcutaneous implantation procedure.

This procedure is similar to the first subcutaneous implant procedure, but in this case the thread has been eliminated. In this procedure an empty catheter 1302 is passed through the subcutaneous space as mentioned above, such that the sharp end comes out through the skin surface on the other end. A vacuum pick-up tool 1304 is passed through the catheter and attaches to one end of a TMO 1306 on the exit end of the catheter (Fig. 13A). Another vacuum pick-up tool 1308 is used to hold the other end of the TMO. Both vacuum pick-up tools are then moved simultaneously such that TMO 1306 is positioned inside the catheter (Figs. 13B and 13C). While the tools still hold the TMO, the catheter is withdrawn such that only the TMO is positioned in the subcutaneous space (Fig. 13D). In this position, the two ends of the TMO optionally extend beyond the skin surface. A scalpel can then be used to make a short slit in the skin at the recipient site, one at each end of the TMO and adjacent to it. The vacuum is then terminated and the pick-up tools disengaged. The protruding ends of the TMO are then grafted into the adjacent slits in the skin of the patient at the recipient site (Fig. 13E), similar to the linear TMO grafting procedure described above.

In this procedure, the grafted TMO sections at the two ends act as markers to the location of the TMO. In addition, the stratum corneum of the skin of the TMO itself forms the channel for the keratin to flow out of the body. As with the thread, the epidermis of the TMO will generate epithelial cells around the keratin of the stratum corneum, such that a stable channel of keratin will form around the stratum corneum of the TMO. The keratin will be secreted through this channel and prevent the formation of inclusion cysts adjacent to the TMO.

Unimplanted micro-organ/TMO material can be stored under cryogenic conditions, for later use, as for example, when the implanted material efficacy is reduced below some required amount.

Alternatively agent can be withdrawn from the nutrient material, purified and injected or otherwise administered to a subject.

### TMO Removal or Neutralization:

An advantage of the micro-organ/TMO for therapy is that the tissue secreting the therapeutic agent is localized at a well-defined location in the body. Therefore, if the treatment needs to be terminated for any reason, simply removing this tissue will stop the delivery of protein. Alternatively the implanted tissue can be ablated or stop functioning as described herein below.

Reference points for visualization of the location of the Micro-organ/TMO are provided by the TMO itself in the case of grafting or by the thread in the case of the subcutaneous implantation or by any other material implanted along with the TMO for this purpose. For example, fluorescent beads may be implanted at each end of the TMO such that a fluorescent light source can be used to locate the beads for the purpose of removing the TMO. Similarly, material that is visible under ultrasound, X-ray, MRI or other visualization source can be used as well as material with magnetic properties.

When grafted, the micro-organ/TMO can be surgically removed with a scalpel dermatome or other cutting means. Instead of removal, the TMO can remain in place but some to all of the cells of the TMO can be ablated with the use of an exterior energy source such as but not limited to laser, cryogenic temperatures, radio frequency and microwave energy. An embodiment of this neutralization procedure involves the introduction of a probe next to the micro-organ/TMO, along the path of the implant on the skin surface. The probe can carry RF or microwave radiation to the area of the TMO, or be cooled to cryogenic temperatures in order to kill the cells of the TMO, with, possibly a small amount of tissue around it.

When implanted subcutaneously, a scalpel or other cutting means may also surgically remove the TMO. For example, a coring device can be used to trace the path of the TMO to remove the implanted tissue with a minimum of surrounding host tissue. The subcutaneous TMO may also be neutralized by ablation with the above mentioned energy sources. In one embodiment, a probe is introduced along the path of the implant. This probe can be used to delivery, for instance, RF energy to cause hyperthermia in the vicinity of the TMO. This will cause significant damage to the majority of the TMO cells such that the protein secretion will cease.

### Examples

### Example 1

### Human skin TMOs, expressing mouse interferon alpha (mIFNα?, implanted in SCID mice.

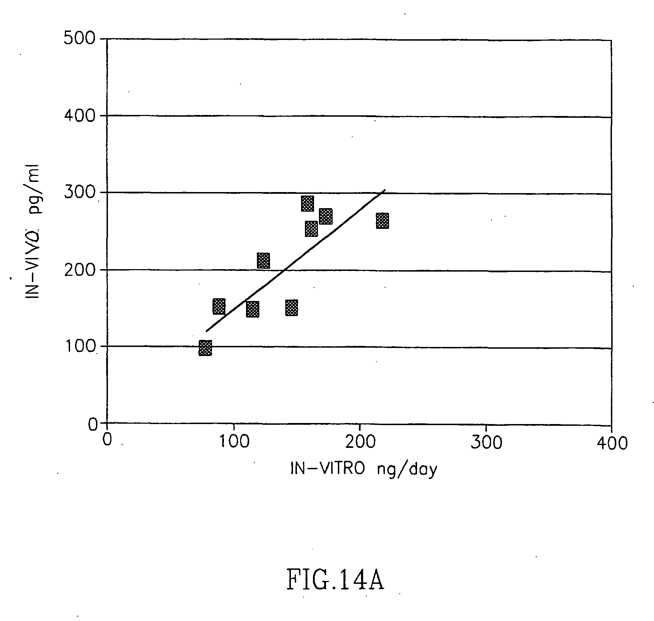

Human skin micro-organs were prepared from fresh skin tissue samples, obtained from tummy-tuck surgery procedure. A section of 1.4-1.5 mm skin thickness (depth) was removed and cleaned using hypochloride solution (10 % Milton solution). A cleaned skin sample was sectioned, using a tissue chopper (TC-2 chopper, Sorval, Du-pont instruments) into 450 micrometer sections (width) under sterile conditions. The resulting micro-organs were placed, one per well, in a 48-well micro-plate containing 400 µl per well of DMEM (Biological Industries - Beit Haemek) in the absence of serum, under 5% CO₂ at 37 °C for 24 hours. Thereafter, each well underwent a transduction procedure in order to generate a therapeutic micro-organ (TMO) using an adeno viral vector (1x10⁹ IP/ml) carrying the gene for mouse interferon alpha (Adeno-mIFNα. Thereafter, the TMOs were again maintained in 400 µl per well of DMEM. The medium was changed every 2-3 days and analyzed for the presence of secreted mIFNα using a specific ELISA kit (Cat. # CK2010-1, Cell Science Inc.). The above-described human skin mIFNα TMOs were implanted sub-cutaneously in several SCID (Severe Combined ImmunoDeficiency) mice. The implanted mice exhibited elevated levels of interferon alpha in their serum for many weeks. The secreted mIFNα detected in these SCID mice serum was found to be biologically active as assayed by a viral cytopathic inhibition assay (data not shown). Fig. 14A shows a correlation analysis between in-vitro secretion of pre-implanted mIFNα-TMOs and the serum in-vivo levels following their implantation. This correlation data indicates that the in-vitro secretion levels, measured prior to implantation, can be used to calculate and dose the amount of TMO that should be implanted in order to achieve a desired therapeutic effect.

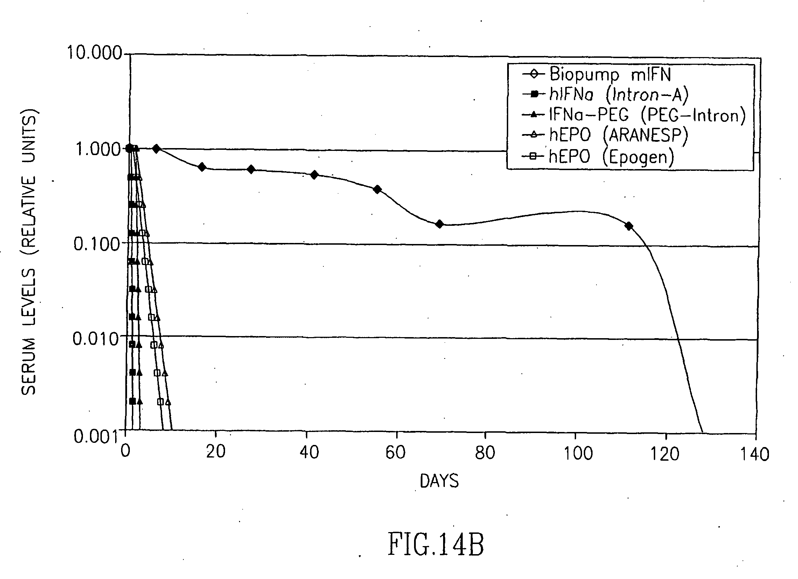

Figure 14B, represents the pharmacokinetics of various injected recombinant therapeutic proteins in a subject, together with mIFNα produced and delivered by human skin TMO in SCID mice. The values represent serum levels of the compared proteins taken from either the label of the injected proteins or from the serum of the SCID mice with the TMO technology, and are expressed as percentage of the respective Cmax for each protein

### Example 2

### Human skin TMOs, expressing mouse interferon alpha (mIFNα), show high reproducibility from patient to patient in protein output

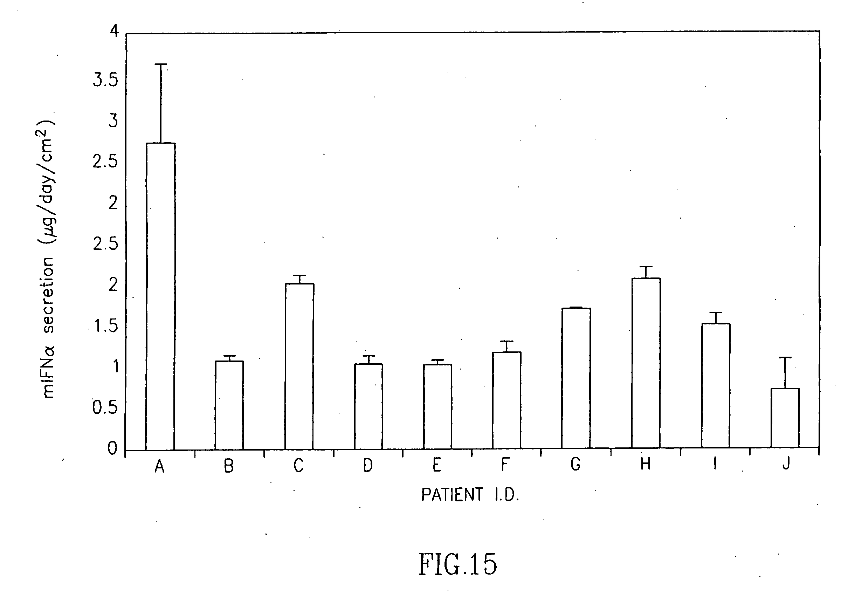

TMOs were prepared and transduced with Ad5/CMV-mIFNα vector using a standard (but non-optimized) protocol, as describe above, including an adeno viral titer of 1x10⁹ IP/ml. Transduction was performed 24 hours post micro-organ preparation. Medium was assayed for in-vitro mIFNα secretion on day 6 following transduction by using a specific ELISA kit (Cat. # CK2010-1, Cell Science Inc.). Fig. 15 shows that the degree of variability between skin samples from different patients, processed at different times, is remarkably small. This low variation between human patients indicates that sufficiently comparable levels of protein secretion can be obtained from a standard sized skin sample taken from patients in practical use for dosing and titrating the amount of TMOs to be implanted in order to achieve the desired therapeutic effect.

### Example 3

### Human skin linear TMOs, expressing human erythropoietin (hEPO), implanted in SCID mice, including re implantation.

Linear (20 mm long and 0.4 micrometer wide) human skin micro-organs were prepared from fresh skin tissue samples obtained from a tummy-tuck surgery procedure. Tissue samples of 0.85-1.1 mm split skin thickness (depth) were removed and cleaned using DMEM containing glutamine and Pen.-Strep in Petri dishes (90 mm).

In order to generate the linear micro-organs, the above tissue samples were cut by a press device using a blade structure as described above, into the desired dimensions: 20 mm x 400 micrometers. The resulting linear micro-organs were placed, one per well, in a 24-will micro-plate containing 500 µl per well of DMEM (Biological Industries - Beit Haemek) in the absence of serum under 5 % CO₂ at 37 °C for 24 hours. Each well underwent a transduction procedure in order to generate a therapeutic micro-organ (TMO) using an adeno viral vector (1x10¹⁰ IP/ml) carrying the gene for human erythropoietin (Adeno-hEPO) for 24 hours while the plate was agitated. The medium was changed every 2-4 days and analyzed for the presence of secreted hEPO using a specific ELISA kit (Cat. # DEP00, Quantikine IVD, R&D Systems).

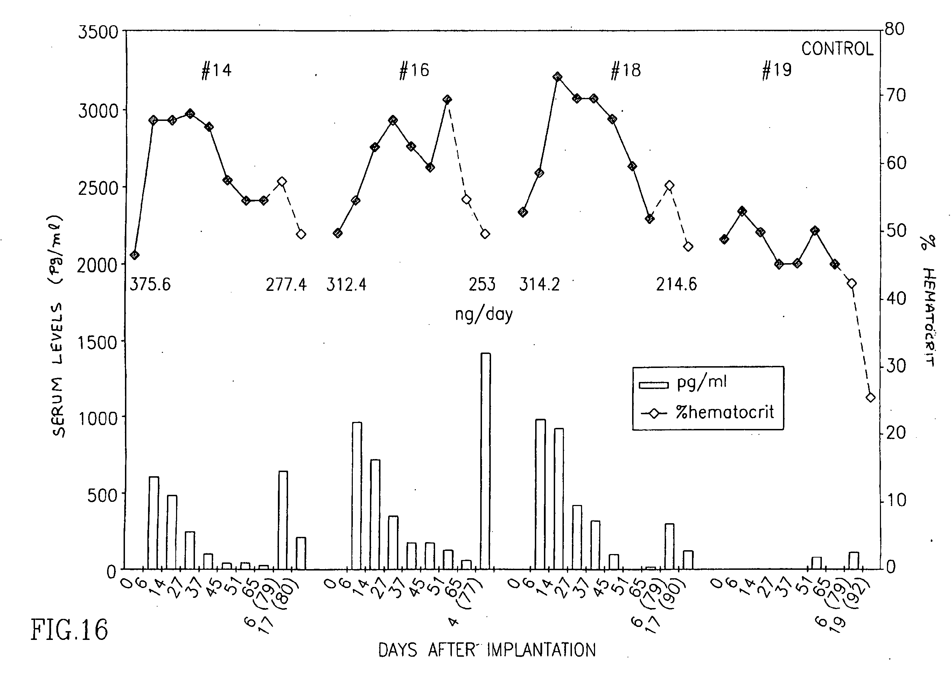

The above described human skin hEPO linear TMOs were implanted sub-cutaneously in several SCID mice. As can be seen in Fig. 16, implanted mouse exhibited elevated levels of erythropoietin in their serum for several weeks. The secreted hEPO detected in these SCID mice serums was found to be biologically active as can be seen by the hematocrite rise. 70 days post implantation, several mice were subjected to a second implantation procedure in which additional Linear hEPO TMOs were implanted, thus achieving a longer hEPO secretion which leads to a longer lasting therapeutic effect.

### Example 4

### Human skin linear TMOs, expressing human erythropoietin (hEPO), implanted in SCID mice in several doses.

Linear (30.6mm long and 0.6 micrometers wide) human skin micro-organs were prepared from fresh skin tissue samples obtained from a tummy-tuck surgery procedure. Tissue samples of 0.85-1.2mm split skin thickness (depth) were removed and cleaned using DMEM containing glutamine and Pen.-Strep in Petri dishes (90 mm).

In order to generate the linear micro-organs, the above tissue samples were cut by a press device utilizing a blade structure as described above, into the desired dimensions: 30.6 mm x 600 micrometers. The resulting linear micro-organs were placed, one per well, in a 24-well micro-plate containing 500 µl per well of DMEM (Biological Industries - Beit Haemek) in the absence of serum under 5 % CO₂ at 37 °C for 24 hours. Each well underwent a transduction procedure in order to generate a therapeutic micro-organ (TMO) using an adeno viral vector (1x10¹⁰ IP/ml) carrying the gene for human erythropoietin (Adeno-hEPO) for 24 hours while the plate was agitated. The medium was changed every 2-4 days and analyzed for the presence of secreted hEPO using a specific ELISA kit (Cat. # DEP00, Quantikine IVD, R&D Systems).

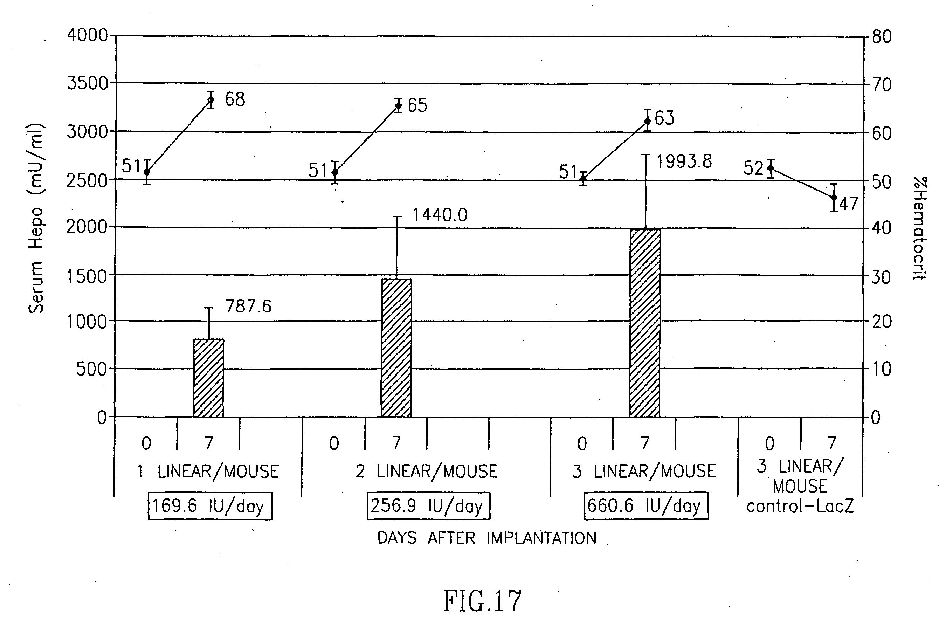

The above described human skin hEPO linear TMOs were implanted sub-cutaneously in several SCID mice in 3 doses (1, 2, 3 linear TMOs per mouse). As can be seen in Fig. 17, implanted mouse exhibited elevated levels of erythropoietin in their serum for several weeks. Furthermore the serum levels found in the various mice are in correlation with the number of implanted linear TMOs, thus achieving a dosage related effect. The secreted hEPO detected in the SCID mice serum was found to be biologically active as can be seen by the hematocrite rise.

### Example 5

### Autologous implantation of miniature swine skin linear TMOs, expressing human erythropoietin (hEPO into immuno competent animals).

Linear (30.6 mm long and 0.6 micrometer wide) miniature swine (Sinclar swine) skin micro-organs were prepared from fresh skin tissue samples obtained from live animals under general anesthesia procedures. Tissue samples of 0.9-1.1mm split skin thickness (depth) were removed using a commercial dermatome (Aesculap GA630) and cleaned using DMEM containing glutamine and Pen.-Strep in Petri dishes (90 mm).

In order to generate the linear micro-organs, the above tissue samples were cut by a press device using a blade structure as described above, into the desired dimensions: 30.6 mm x 600 micrometers. The resulting linear micro-ograns were placed, one per well, in a 24-well micro-plate containing 500 µl per well of DMEM (Biological Industries - Beit Haemek) in the absence of serum under 5 % CO₂ at 37 °C for 24 hours. Each well underwent a transduction procedure in order to generate a miniature swine skin therapeutic micro-organ (pig skin-TMO) using an adeno viral vector (1x10¹⁰ IP/ml) carrying the gene for human erythropoietin (Adeno-hEPO) for 24 hours while the plate was agitated. The medium was changed every 2-4 days and analyzed for the presence of secreted hEPO using a specific ELISA kit (Cat. # DEP00, Quantikine IVD, R&D Systems).

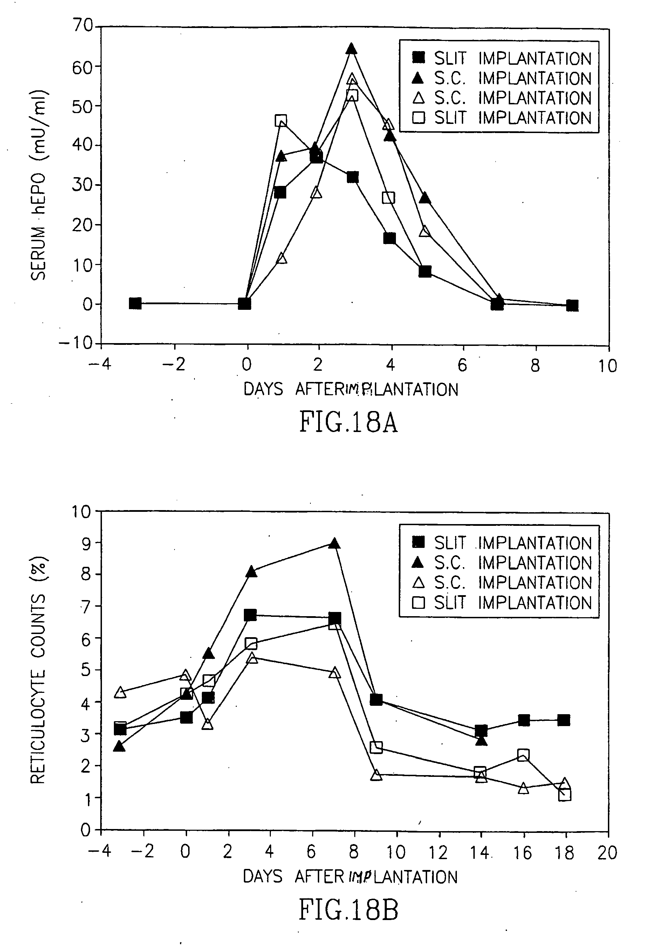

The above described miniature swine skin hEPO linear TMOs were implanted both subcutaneously and grafted as skin grafts in several immune competent miniature swines (in two of the miniature swine, the TMOs-hEPO were implanted subcutaneously, and in two different miniature swine, TMOs-hEPO were grafted in 1mm deep slits). Sufficient number of TMOs-hEPO were implanted in each miniature swine so that their combined pre-implantation secretion levels in each pig was approximately 7 micrograms per day. Elevated serum hEPO levels (Figure 18A) determined by an ELISA assay and reticulocyte count elevation (Figure 18B) were obtained for seven days after implantation. Figures 18A and B indicated the delivery of therapeutic quantities of physiologically active (erythropoietic effect) hEPO into the pig serum.

### Example 6

### Human skin linear and Mesh TMO's expressing human erythropoietin (hEPO) in-vitro

Linear (28 mm long and 0.6 micrometer wide) and Mesh (0.6 micro-meter wide of each mesh segment) human skin micro-organs were prepared from fresh skin tissue samples obtained from a tummy-tuck surgery procedure using a commercial dermatome. Tissue samples of 0.85-1.2 mm split skin thickness (depth) were removed and cleaned using DMEM containing glutamine and Pen.-Strep in Petri dishes (90 mm).

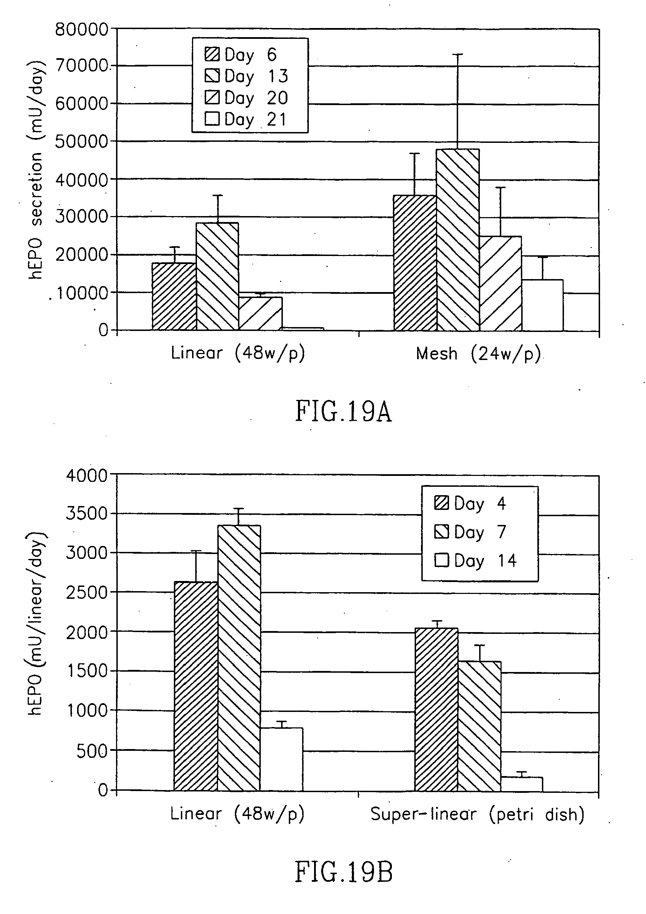

In order to generate the linear and the Mesh micro-organs, the above tissue samples were cut by a press device using the blade cassette described in Fig. 4A for generating linear micro-organs or the blade cassette described in Fig. 8 for generating a Mesh micro-organ. The resulting linear/mesh micro-ograns micro organs were placed, respectively, one per well, in a 48/24-well micro-plate containing 500/1000 µl per well of DMEM (Biological Industries - Beit Haemek) in the absence of serum under 5 % CO₂ at 37 °C for 24 hours. Each well underwent a transduction procedure in order to generate a therapeutic micro-organ (TMO) using an adeno viral vector (1x10¹⁰ IP/ml) carrying the gene for human erythropoietin (Adeno-hEPO) for 24 hours while the plate was agitated. The medium was changed every 3-4 days and analyzed for the presence of secreted hEPO using a specific ELISA kit (Cat. # DEP00, Quantikine IVD, R&D Systems). As can be seen in Fig. 19A, hEPO protein was detected for in-vitro secretion for 31 days after transduction.

### Example 7

### Human skin linear and Super linear TMO's expressing human erythropoietin (hEPO) in-vitro

Linear (20 mm long and 0.6 micrometer wide) and portions of super linear (15 mm long and 0.6 micrometer wide) human skin micro-organs were prepared from fresh skin tissue samples obtained from a tummy-tuck surgery procedure. Tissue samples of 0.85-1.2 mm split skin thickness (depth) were removed and cleaned using DMEM containing glutamine and Pen.-Strep in Petri dishes (90 mm).

In order to generate the linear and the super linear micro-organs, the above tissue samples were cut by a press device using the blade cassette described in Fig. 4A for generating linear micro-organ or the blade cassette described in Fig. 5A for generating a super linear micro-organ. The resulting linear/super micro organs were placed, one per well (linear) containing 500 ul DMEM or in a petri dish containing 3750 ul of DMEM. (Biological Industries - Beit Haemek) in the absence of serum under 5 % CO₂ at 37 °C for 24 hours. Each well underwent a transduction procedure in order to generate a therapeutic micro-organ (TMO) using an adeno viral vector (1x10¹⁰IP/ml) carrying the gene for human erythropoietin (Adeno-hEPO) for 24 hours while the plate was agitated. The medium was changed every 3-4 days and analyzed for the presence of secreted hEPO using a specific ELISA kit (Cat. # DEP00, Quantikine IVD, R&D Systems). As can be seen in Fig. 19B, hEPO protein was detected for in-vitro secretion for 14 days after transduction.

### Example 8

### Human skin linear TMO's, derived from a skin sample harvested with new dermatome, expressing human erythropoietin (hEPO) in-vitro

Linear (30.6 mm long and 0.6 micrometer wide) human skin micro-organs were prepared from fresh skin tissue samples obtained from a tummy-tuck surgery procedure. Tissue samples of 0.9-1.1 mm split skin thickness (depth) were removed using the dermatome described with respect to Figs. 3A-3E, and cleaned using DMEM containing glutamine and Pen.-Strep in Petri dishes (90 mm).

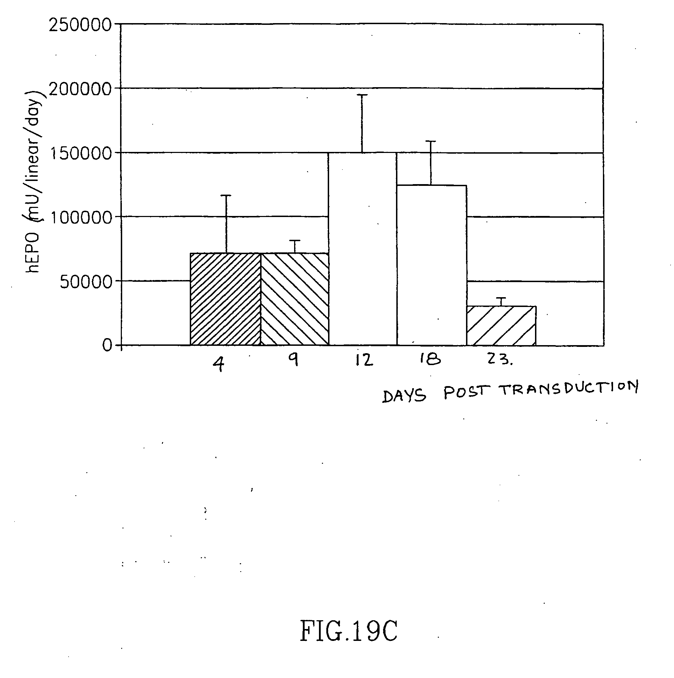

In order to generate the linear micro-organs, the above tissue samples were cut by a press device described by the present invention using the blade cassette described in Fig. 4A for generating linear micro-organs The resulting linear micro-ograns were placed, one linear segment per well containing 500ul DMEM (Biological Industries - Beit Haemek) in the absence of serum under 5 % CO₂ at 37 °C for 24 hours. Each well underwent a transduction procedure in order to generate a therapeutic micro-organ (TMO) using an adeno viral vector (1x10¹⁰ IP/ml) carrying the gene for human erythropoietin (Adeno-hEPO) for 24 hours while the plate was agitated. The medium was changed every 3-4 days and analyzed for the presence of secreted hEPO using a specific ELISA kit (Cat. # DEP00, Quantikine IVD, R&D Systems). As can be seen in Fig. 19C, hEPO protein was detected for in-vitro secretion for 23 days after transduction.

### CLOSED STERILE MICRO-ORGAN PROCESSING CASSETTE

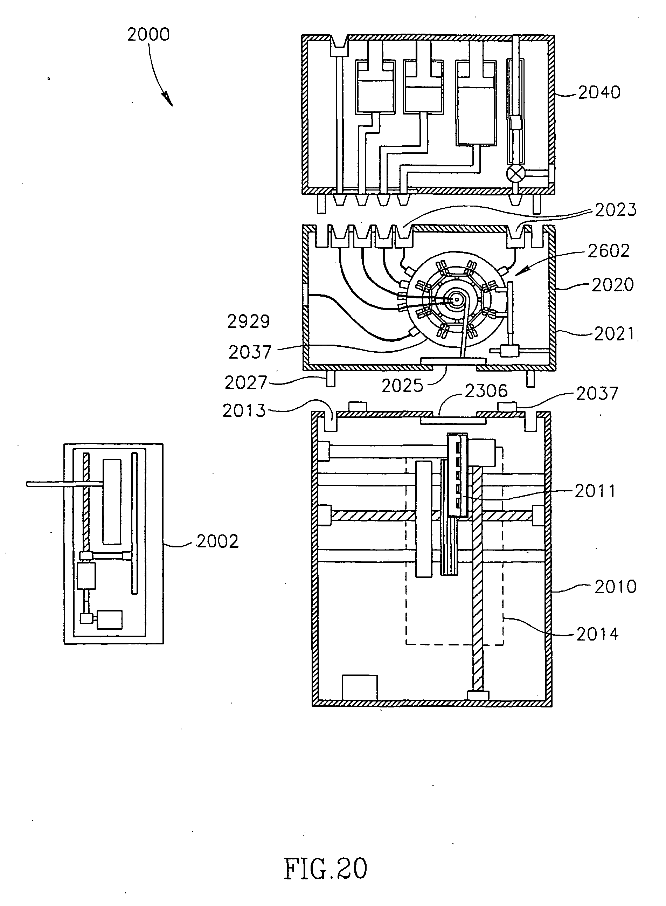

Figs. 20-39 describe cassette modules that are used for processing all of the stages of micro-organ/TMO processing starting from tissue harvesting and through implantation in a subject. In the described cassette modules, various functions described above are performed in a sterile environment, with transfer of micro-organs/TMOs between modules performed in an efficient, sterile and controllable manner.

Fig. 20 shows the main cassette modules 2000, with the modules separated for ease of visualization. The main modules are a tissue harvester 2002, a micro-organ module 2010, a bio-processing module 2020, and a fluidics module 2040. Each module comprises a plastic or other bio-compatible housing. In general, tissue harvester 2002 is detached from the rest of the cassette when tissue is being harvested, and is then attached to micro-organ module 2010 to transfer the harvested tissue thereto. Each set of modules is unique to a given subject and a given sample, identified by such means as bar codes. After use, the modules are preferably discarded.

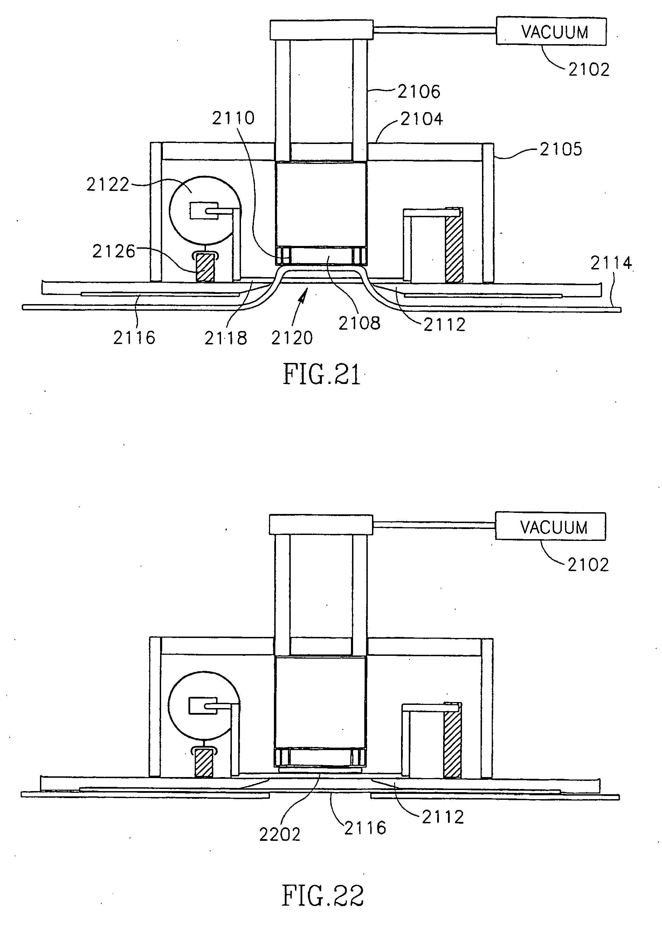

Figs. 21 and 22 show the operation and details of harvester 2002.

In a clinically sterile environment such as an outpatient clinic or operating room, a skin sample is taken from the subject using skin harvester 2002. Harvester 2002 is optionally powered by battery either on board or in a separate power module (not shown), but may also be powered by means such as medically isolated power supply.

In accordance with the design principles of the tissue harvesting apparatus as described above, with respect to Fig. 3, harvester 2002 uses a vacuum source 2102, which can either be a dedicated, portable vacuum source or derived from non-mobile installed vacuum source. Standard surgical site preparation is made at the donor site, and local anesthetic administered.

A port 2116 is opened on a base plate 2412 to form a window 2120, which is the only opening to the sterile tissue harvester. It is then mounted onto a subject at a desired location, with means (not shown) to provide sufficient pressure so as to cause a skin surface 2114 to bulge through window 2120.

A plunger 2106 is lowered through sterile bushings 2104 in a sealed housing 2105 as needed to contact skin, and vacuum is applied through a sample carrier 2108 via access holes 2110 to hold the surface of the skin flat against the surface of the sample carrier.

The contact surface of the sample carrier is positioned vertically by the plunger and maintained at a desired distance above the cutting edge of the blade, in order to cut the desired thickness of skin sample.

A blade 2118 (shown in an end on view) is optionally oscillated side to side by a motor 2122, which is driven forward, for example, by a screw drive 2126 to cut the tissue (e.g., skin). Drive 2126 is actuated by a motor (not shown).

Figure 22 shows a resulting harvested skin sample 2202 attached to the carrier, with port 2116 in the closed position. The tissue harvester module is now sealed again in a sterile manner and ready for transport to and transfer of the micro-organ to the micro-organ module.

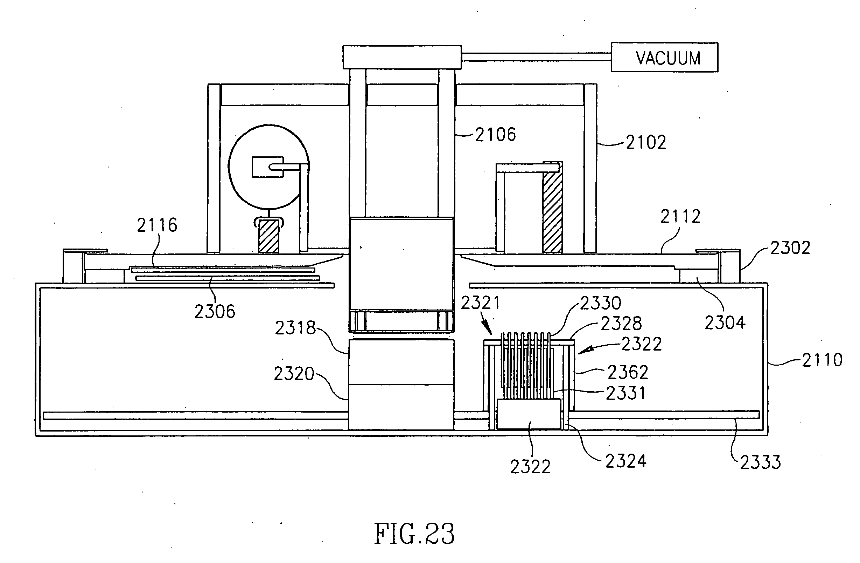

Figs. 23-25 illustrate the formation of a micro-organ from a skin sample.

Closed harvester 2002 with skin sample on the carrier, is then detachably mounted via air tight gasket 2304 to micro-organ module 2010 via clips 2302 as shown in Fig. 23. A top view of the placement location of the harvester module is shown at 2014 in Fig. 20.

In micro-organ module 2010, a trimming cartridge 2320 comprises typically two parallel blades 2318 spaced at the desired length of individual segments of the superlinear micro-organ, typically in the range of 30 mm, and supported by base 2321. Optionally, trimming cartridge 2320 comprises four blades forming a rectangle, that delineates both dimensions of length and width of the sample.

Trimming cartridge 2320 is aligned with the sample carrier of harvester 2002, and ports 2116 on the harvester and 2306 on the micro-organ module, are opened.

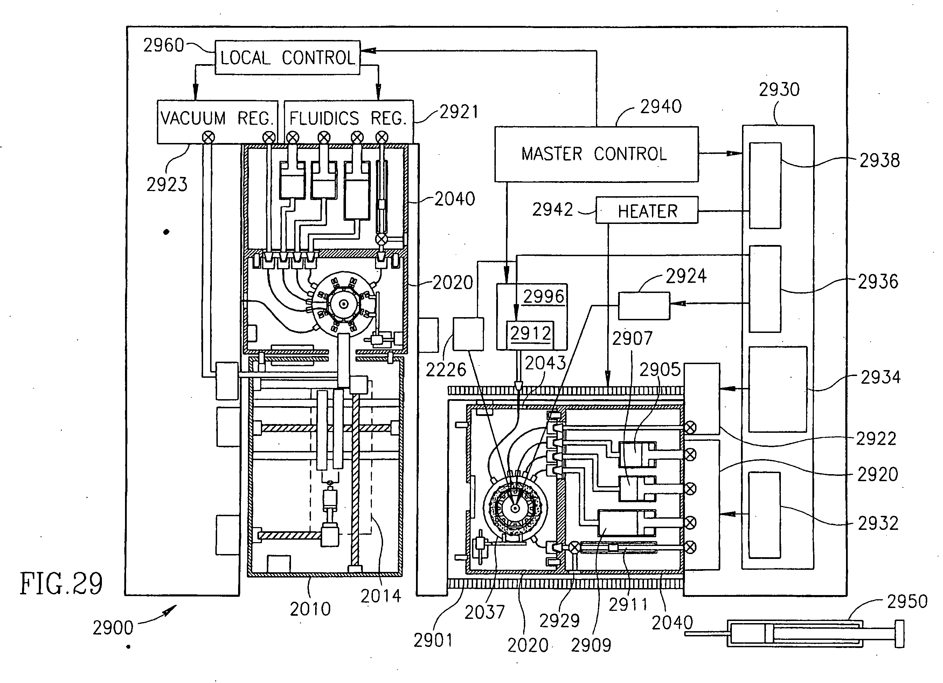

A wetting agent used to keep the tissue sample moist during the cutting and transfer processes, is delivered to both the trimming and cutting cartridges via a dispenser (not shown in Fig. 23, but can be seen in a top view in Fig. 29).

Plunger 2106 is driven against cartridge 2320, causing the blades to cut through the skin to the carrier beneath, thus trimming two edges of the skin sample.

Plunger 2106 is retracted to a height just above blades 2318, while the vacuum is maintained, thus holding both the trimmed sample and cut margins against the carrier.

In accordance with the principles disclosed above with respect to Figs. 4 and 5 a cutting cartridge 2321 comprises a stack of parallel cutting blades 2330 spaced apart by spacers 2331 mounted in support base 2322, and arranged so that odd numbered blades are displaced longitudinally against their even numbered counterparts typically by a distance equal to the width of an micro-organ, typically in the range of a few hundred micrometers.

A removal mask 2328 has been inserted between blades 2330, and is held in place by a bracket 2326, which rides against an offset 2324. Bracket 2326 is optionally latched in position against pressure such as a compressed spring, held in place by a latch (not shown).

Trimming cartridge 2320 and micro-organ cartridge 2321 are driven by a screw drive 2233 so that micro-organ cartridge 2321 is now aligned with the carrier.

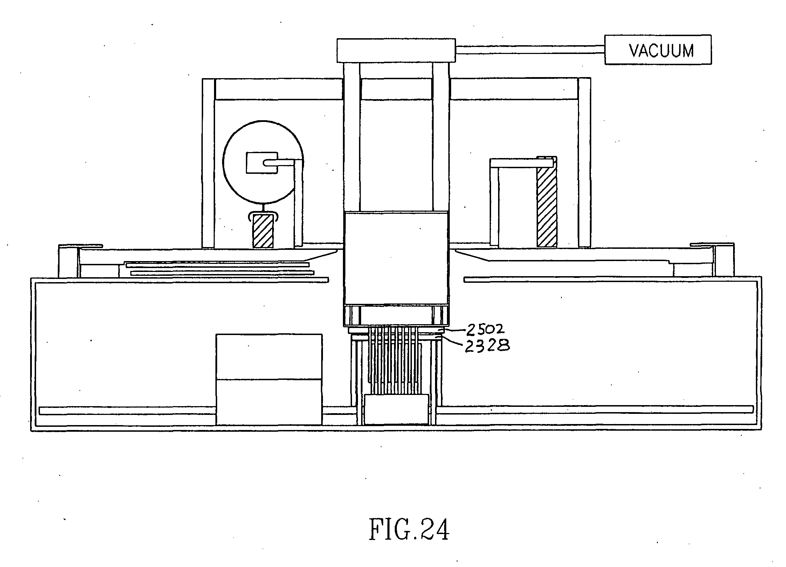

Plunger 2106 is driven against blades 2330, as shown in Fig. 24 until they cut through the skin to the carrier beneath.

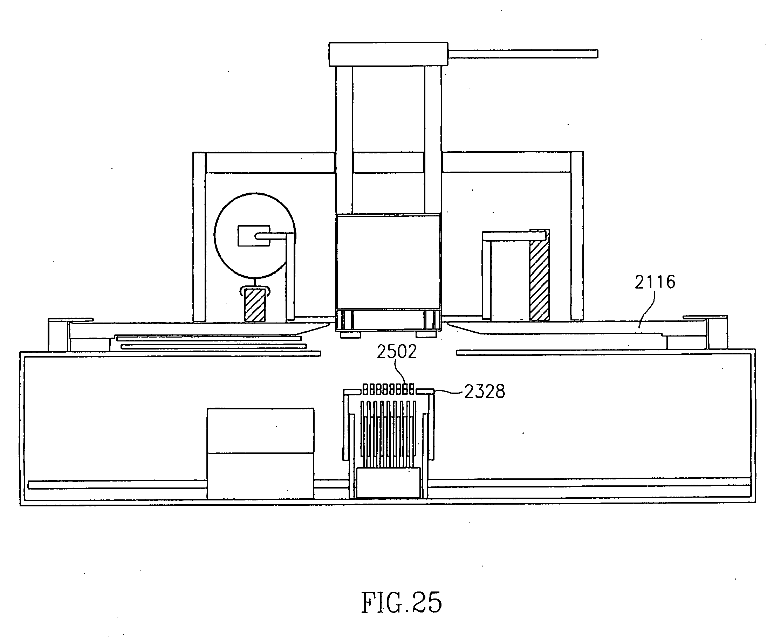

Plunger 2306 is raised, typically back to its initial starting position prior to mounting on 2010, as shown on Fig. 25.

Mask 2328 is raised up above blades 2330 by bracket 2326, which is lifted by one of several means, such as by recoil of compressed spring, actuated by release of a latch holding it (not shown). The latch release can be actuated by the pressure from the plunger during the cutting by the cutting cartridge, among others.

A resulting super-linear micro-organ 2502, rests on top of mask 2328, in a known position and orientation ready for transport. Note that carrier 2108 now holds the tissue margins that were trimmed, leaving behind the superlinear micro-organ on the mask.

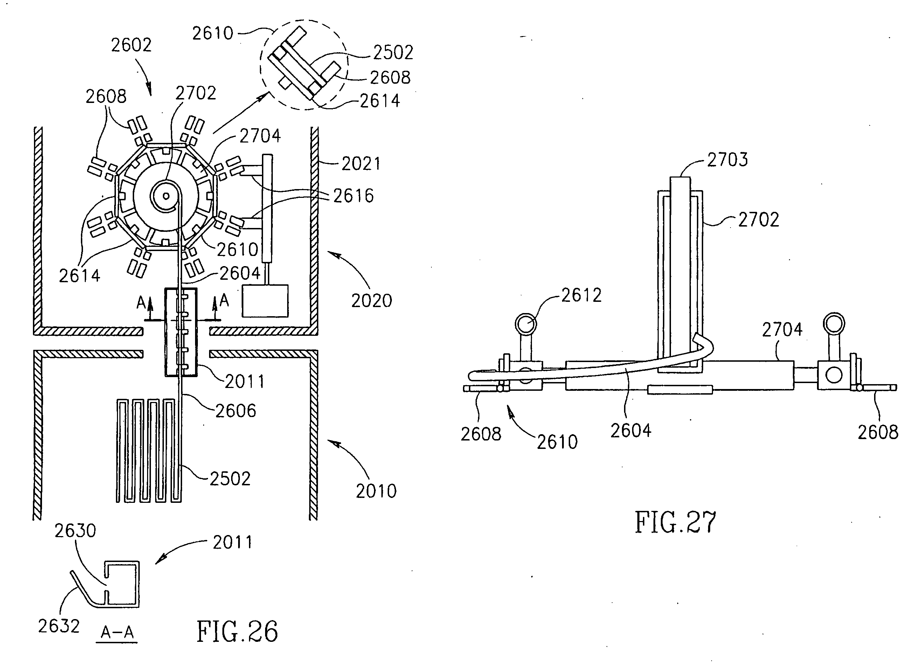

Figs. 26-28 show some details of a bio-processing module 2020, which is also shown in Fig. 20, to which further reference is now made and the transfer of a micro-organ thereto. Bio-processing module 2020 comprises a housing 2021 having a port 2024 formed therein. Within housing 2020, a mounting mechanism 2602 is rotatably mounted, as described below. Housing 2020 is also formed with a plurality of fluidics ports 2023, which transfer power to elements within module 2020 from fluidics module 2040. Housing 2020 is also formed with mounting pins 2027 which mate with matching holes 2013 in the housing of module 2010 and hermetically seal the two modules with the aid of sealing gaskets 3037.

Also shown in Fig. 20 is a vacuum guide 2011, which is shown withdrawn into module 2010.

Details of mounting mechanism 2602 are shown in Figs. 26 and 27. Mechanism 2606 comprises an inner rotating mechanism 2702 and a rotating micro-organ holder 2704. Attached to inner rotating mechanism is a vacuum pick-up lead 2604, which is released by rotating the inner rotating mechanism.

Fig. 26 shows, schematically, the transfer of the micro-organ between modules 2010 and 2020. Ports 2025 and 2306 have been opened (and are not shown). Vacuum guide 2011 has a starting position in the port area between the modules and vacuum pick-up lead 2604 is resting on vacuum guide 2011 so that its pick-up 2606 position is positioned slightly away from micro-organ 2502 which is resting on mask 2328. In order to grasp micro-organ 2502, inner rotating mechanism 2702 is rotated clockwise slightly so as to push the pick-up 2604 adjacent to the side of micro-organ 2502 and overlaps its end. Vacuum pick-up 2606 is activated and the pick-up lead is withdrawn into module 2020, by rotating inner mechanism 2702 counterclockwise and being guided over vacuum guide 2011 which has only enough vacuum pressure so as to keep the vacuum pick-up lead 2604 and the micro-organ 2502 after it clinging to it. When the leading end of the micro-organ reaches the rotating micro-organ holder 2704, it lies on a segment mount 2610. Segment mount 2610 (shown magnified in the blow-up circle) comprises at least two closure portions 2608, an optionally expandable cross-bar 2614 (described below) and also optionally comprises an eye 2612, whose function is described below. Once the micro-organ reaches micro-organ holder 2704, the inner rotating mechanism 2702 locks onto the outer rotating mechanism 2703 such that both turn together as a unit with the effect that the micro-organ holder 2704 rotates counter- clockwise, loading the micro-organ onto it.

Vacuum guide 2011 applies a low level vacuum to the micro-organ so that the organ remains oriented and does not twist during transport. Optionally, it is in the form of a rectangular tube (shown in insert A-A) formed with holes 2630 along its length, through which a slight vacuum is applied, enough for the micro-organ to slide along it as it gently clings to its side. Optionally an alignment member 2632 is attached, which aligns the micro-organ and prevents curling. The trough also keeps lead 2604 positioned in guide 2011.

Rotating micro-organ holder 2704 is provided with a series of segment mounts, 2610 (shown magnified in the blow-up circle), each the length of a single segment of the micro-organ, on which the clips 2608 are located, one on each end of the mount. Thus, as the micro-organ holder rotates, one micro-organ segment is laid on a clip on one end of the segment mount and then another end is laid on a clip on the other end of the segment mount. As the segment mounts pass a closure mechanism 2616, the mechanism rotates so as to lift two paddles that close clips 2608 on the ends of the segment mount. Once the micro-organ is completely held, the vacuum of the vacuum pick-up and the vacuum guide can be released. With micro-organ 2502 securely mounted on micro-organ holder 2704, guide 2011 is then retracted into micro-organ cassette 2010, actuated by a screw drive, until it clears the ports, which are then closed. The micro-organ module 2010, along with the tissue harvester module 2202 may then be discarded.

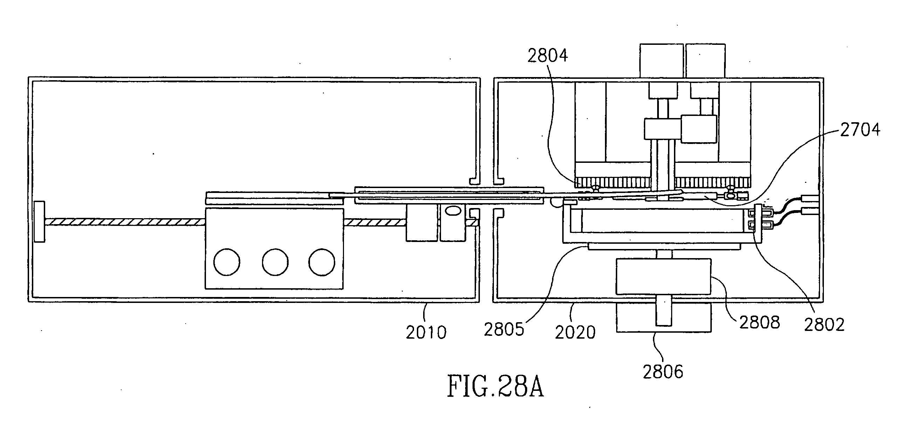

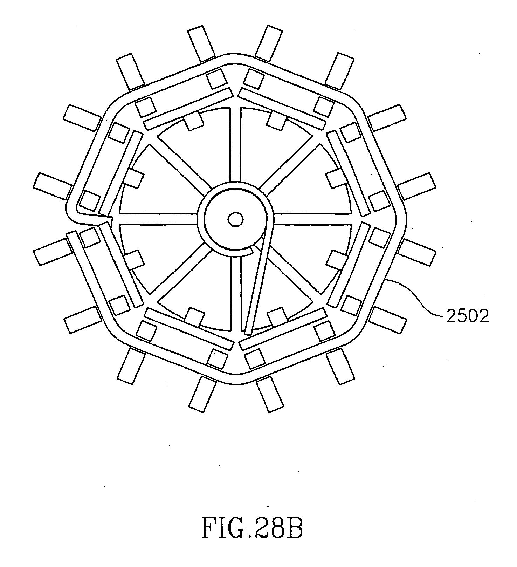

Fig. 28A shows a side view of the above process and Fig. 28B shows the micro-organ mounted on the segment mounts.

A bio-reactor base 2802 is raised until the super-linear micro-organ holder 2704 is seated against the inner surface of base 2802. Base 2802 is raised, for example, via a support plate 2805 driven by motor 2806 through coupling 2808. In one embodiment the base with the super-linear micro-organ within it is not covered with a cover. Optionally, a cover could be applied to prevent splashing of the fluid within the bioreactor due to agitation or to decrease evaporation, if needed. A cover could be a loosely fitting cover over the base as in a petri-dish, or it could be a hermetically sealed cover to entire seal the bioreactor. The cover can either be made of a hard plastic material or other biocompatible material, or it could be made of a membrane, such as a gas permeable, liquid impermeable membrane or other type of membrane. A gas permeable membrane would have the added advantage of allowing for control of the gaseous environment by means of the gas concentration in an additional chamber sounding said bioreactor. The cover could either be beneath the blade assembly 3220 (described below) or above it.

Fig. 29 shows a processing station 2900, having a series of modules mounted in it. In an exemplary embodiment of the invention, the functions described above with respect to Figs. 23-28 are performed when the modules are parked in the left hard portion of control module 2900, as shown. Clearly shown are modules 2010, 2020 and 2040. Module 2002 is connected at a port marked as 2014 in Figs. 20 and 29, on module 2010. In operation, modules, 2002, 2010, 2020 and 2040 are hooked up to a vacuum regulator 2923 and a fluidics controller 2921, for example, by quick disconnects. Vacuum regulator 2923 and a fluidics controller 2921 are under the control of a local controller 2960 which is in turn under the control of master control 2940. Local controller 2960 also controls motors necessary for opening ports rotating holders, etc., as described above. It should be understood that while a mix of fluidics and electrical (motor) control is described, only fluidic or only electrical control can be used.

After the tissue sample has been harvested by harvester module 2002, harvester module 2002 is linked to module 2010, via port 2014. The harvested tissue sample is cut (Figs. 23-25) and the cut micro-organ is transferred to module 2020 as described with respect to Figs. 26-28. At this point, module 2010, with module 2002 still attached to it, is no longer needed and can be disconnected from module 2020 and discarded.

Bio-processing module 2020 and fluidics module 2040 are then transferred to the docking station on the right side of Fig. 29 in the shown orientation. A plurality of docking stations can be provided in the processing station 2900, for cassettes containing different tissue samples, which may be produced from one of a number of patients/sites. All of the cassettes in the plurality of docking stations need to pass through the dock on the left at the commencement of their processing. On this side of the figure, modules 2020 and 2040 are shown attached to a fluidics actuator 2920 (controlled by a fluidics controller 2932) and a vacuum regulator 2922 (controlled by a vacuum controller 2934). Motors 2224, 2226, 2906 and 2912 are actuated by motor control 2936.

Modules 2020 and 2040 are placed in an envelope 2901 and are kept at a desired temperature by a heater 2942 responsive to a temperature sensor (not shown) and controlled by a heater controller 2938. Controllers can be separate controllers or can be part of a large local controller 2930. Local controller 2930 also controls sampling and analysis from the TMOs via sampler 2912, which samples fluids from bio-reactor 2037 via a sterile port 2943 such as a septum and feeds them to analyzer 2996, which communicates with master control 2940. Sensor can optionally sense a plurality of parameters such as temperature, humidity, CO₂, pH or other commonly monitored parameters used in bioreactors for documentation or control.

Fluids, such as nutrients, waste products, gases, etc., are transferred to and from bio-reactor 2037 by fluidics module 2040.

Growth medium is stored in dispensing volume 2905 and delivered to bioreactor 2037 under control of fluidics controller 2932.

Waste medium is removed into dispensing volume 2909 under control of fluidics controller 2932,

Dispensing volume 2907 can deliver sterile gases such as oxygen, nitrogen, CO₂ or mixture of them. Alternatively, volume 2907 can be used to deliver antibiotics, disinfectant or other desired fluid.

Sterile air filters (not shown) can be added to each of the modules if needed to allow for equilibration of air pressure during application of vacuum.

Under control of a master TMO processing algorithm executed by master controller 2940, a time sequence of steps is followed involving introduction and removal of fluids, including gene transfer vector at appropriate times, and agitation by means such as rotational and translational movement of the micro-organ mount in bioreactor 2037 or by the use of accoustic energy applied to the fluid in the bioreactor 2037 or by other means. The timing and duration of these steps are determined, for example, either by preset program and/or as determined by measured process conditions, and is typically selected to match the properties of the specific gene or gene transfer vector, the intended application, and certain data from the subject.

Gene transfer vector dosing volume 2950 is normally maintained at cryogenic temperatures by means not shown, and at the appropriate time is removed and thawed, and administered via a sterile port 2929, such as a septum, and fills dispensing volume 2911, which in turn is delivered to bioreactor 2937 under control of fluidics controller 2932.

At an appropriate time, typically 24 hours after formation of the super-linear micro-organ, gene transfer vector is injected through sterile port 2929 to fill dispensing volume 2911, and the delivery of the first portion of said vector is commenced.

### Assaying TMO performance:

Assaying the performance parameters of the TMOs, such as protein production rate, can be done at various times before, during, and after application of the gene transfer vector, in order to monitor and adjust the preparation process so as to result in TMOs having performance in a desired range, such as producing a desired range of protein per unit time. One way such assaying can be performed is by means that require a sample of the tissue or surrounding fluid that is physically removed from the bioreactor, such as immunoassay or similar chemical or biological assays which use up a portion of sampled material. Another way, which could be used instead of or in combination with the first, is by means that can sense the performance parameter of the TMOs or their medium in the bioreactor without requiring physical removal, such as optical means, molecular probe sensor technology such as DNA or protein arrays, or others known in the art. Either way, TMO performance can be assayed at various time points from the time the microorgans are first introduced to the bioreactor until they are removed for use.

The protocol used to control the conversion micro-organss of a given subject into TMOs can utilize one or more variables during the preparation of the TMOs in order to reach the desired performance range, examples of such variables including but not limited to:
1. Number of vector treatments: one or more exposures of the micro-organs to gene transfer vector by addition to the bioreactor medium containing the micro-organs.
2. Duration of each treatment: each exposure typically ends by replacement of some or all the medium to remove remaining vector from the bio-reactor, though it can also achieve reduced vector activity in the bioreactor by simply allowing it to deactivate with time, or by heating to deactivate, or other such means. The time in between exposures is also a variable.
3. Dose of vector used: each exposure utilizing a specified dose or amount of gene transfer vector, which may be chosen to be the same or different for the various exposures. The vector amount to be applied may typically be varied by using the same or different specified potency of vector (titer of infectious and non-infectious viral particles, in the case of a viral vector), or by varying the total volume amount added to the bioreactor.
4. Vector enhancement means: Vector action may optionally be enhanced by using one or more means to increase efficiency of gene transfer whether while the vector is present in the medium of the micro-organs, or before or after it. Such means include by way of example: addition of various forms of chemical agents known to enhance vector uptake or effect; physical treatment of the micro-organs such as abrasion or perforation of the tissue (such as stratum corneum or dermis in the case of skin) to enhance entry of vector; physical agitation of the micro-organs within their medium; causing physical vibration of the micro-organs or their medium; exposure of micro-organs or their medium to sonic or ultrasonic energy; utilization of various electrical means to enhance vector uptake and effect such as electroporation and application of electromagnetic fields, among others.
5. Adjustment of maintenance conditions: The scheduled amounts and timing of medium removal and replacement, the rate of gas exchange, the addition of agents such as buffer and other chemicals to the bioreactor medium, in order to maintain the desired condition of the growth medium in the bioreactor.
6. Scheduling of steps: The timing and duration of each step in the conversion from micro-organ to TMO, from the time the micro-organs are prepared until TMOs are ready for use.

The algorithm used to prepare TMOs can be a preset, fixed sequence of specific steps of known timing and duration, involving fixed preset values for the aforementioned variables and schedule of steps. Alternatively, the algorithm can be adaptive, designed to self adjust one or more of the variables above based on the measured TMO performance at various stages in the preparation, in order to alter their performance in order to reach the desired value range at the time for use in treating a subject.

During the process, samples of the medium in bioreactor 2937 are taken by sampler 2912 and analyzed by analyzer 2996, typically to quantify the amount produced of the specific desired protein, using one of the analytical methods known in the art, such as ELISA. Other tests may also be used to characterize the protein produced by the TMO, such as spectral analysis or other tests.

In addition, sampling is typically used for testing safety aspects of the TMOs, such as sterility and freedom from certain adventitious agents.

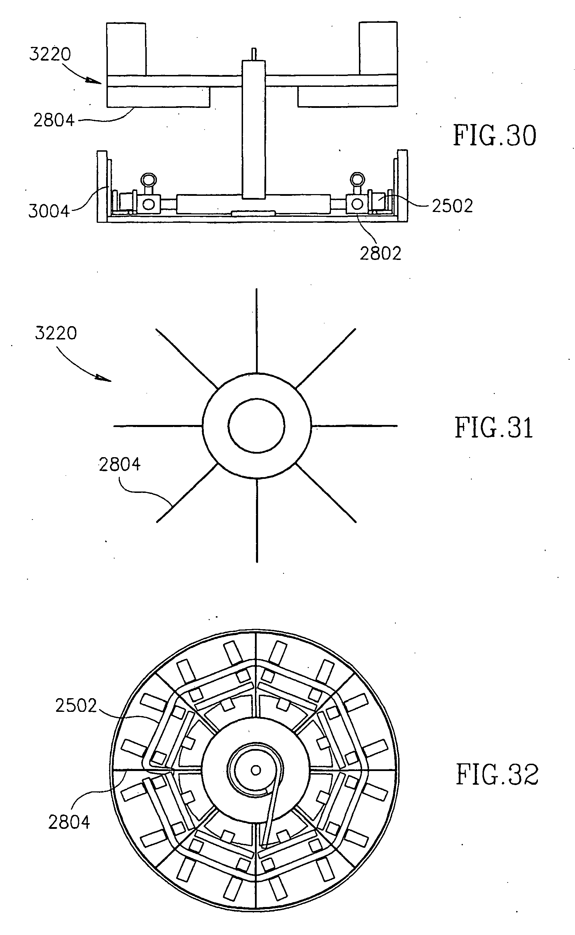

When the process results indicate the TMOs are ready for administration to a subject, base 2802 is further raised (as shown in Fig. 30) to drive it into blades assembly 3220, whose blades 2804 fit in the preformed slots between adjacent segment mounts (Fig. 32). Fig. 31 shows a view of the blades from above (as compared to the side view of Fig. 30). Upon reaching the base, blades 3804 cut the superlinear TMO into individual segments.

Optionally, blades assembly 3220 can be left in place and used to substantially separate the fluid and the individual micro-organs/TMOs into separate chambers. This can be used to enable individual sampling from each individual cut micro-organ/TMO segment. In this instance, base 2802 is lined along its bottom and sides with a soft, biocompatible, impermeable layer 3004, such as silicon rubber, and blades assembly 3220 has an inner disk 3012 of the same material embedded on its lower side. Blades 3220 fit snuggly between the inner diameter of base 2802, and upon lowering, cut into layer 3004, while the disk 3012 mates firmly against the bottom of base 2802. The result is the creation of an individual chamber for each TMO segment, substantially isolated from the other segments, allowing for measurement of individual secretion levels from each segment.

To prepare TMOs for administration to the subject, the requisite number of segments to be administered is estimated, typically using as inputs such data as, but not limited to:
a) Corresponding amounts of the same therapeutic protein routinely administered to such subjects based on regulatory guidelines, specific clinical protocols or population statistics for similar subjects.
b) Corresponding amounts of the same therapeutic protein specifically to that same subject in the case the he/she has received it via injections or other routes previously.
c) Subject data such as weight, age, physical condition, clinical status.
d) Pharmacokinetic data from previous TMO administration to other similar subjects.
e) Response to previous TMO administration to that subject.

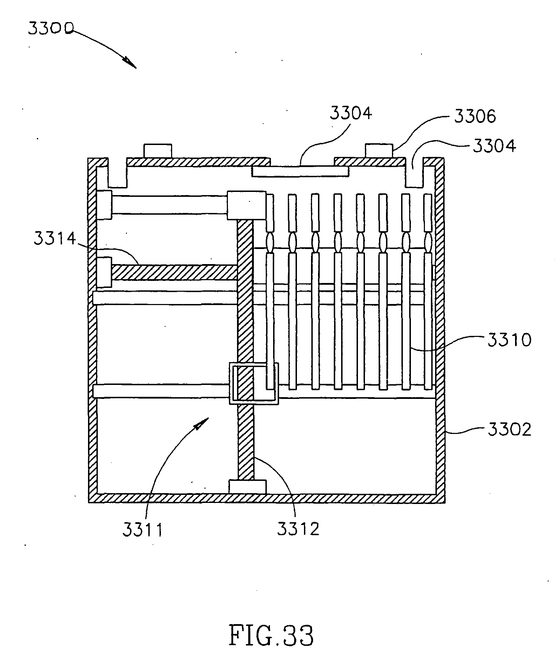

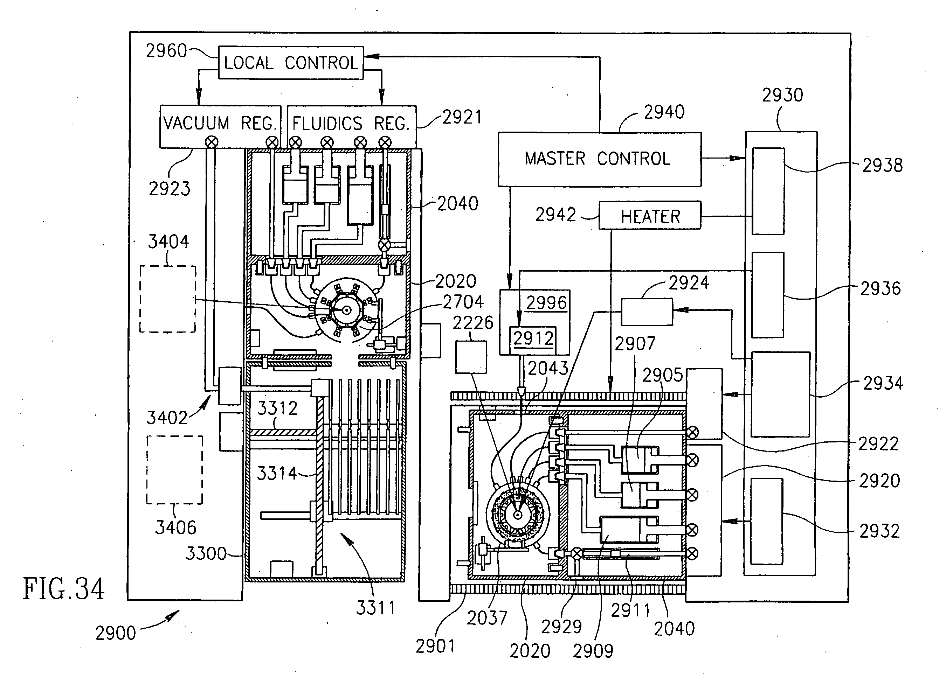

The modules are removed from docking station, and a TMO transfer module 3300 is detachably but hermetically attached to bio-processor 2020 via connects 3304 and sealing gasket 3306 (Fig. 33), and inserted back into the docking station on the left side of the processing station 2900 (Fig. 34).

As shown in Fig. 33 and 34, transfer module 3300 comprises a housing 3302, having a port 3305 fitted therein, a plurality of transfer pins 3310 mounted on an x-y stage 3311 comprising two lead screws 3312 and 3314, driven by motors 3406 (shown schematically), transfer pins 3310 being adapted for selective passage through port 3305.

As shown in Fig. 34, module 2020 has been joined to module 3300, while remaining joined to module 2040, to form assembly 3402. The ports between modules 2020 and 3300 are opened. Two motors, 3404 and 3406 are shown very schematically. These motors are operative to rotate micro-organ holder 2704 and to operate x-y stage 3311.

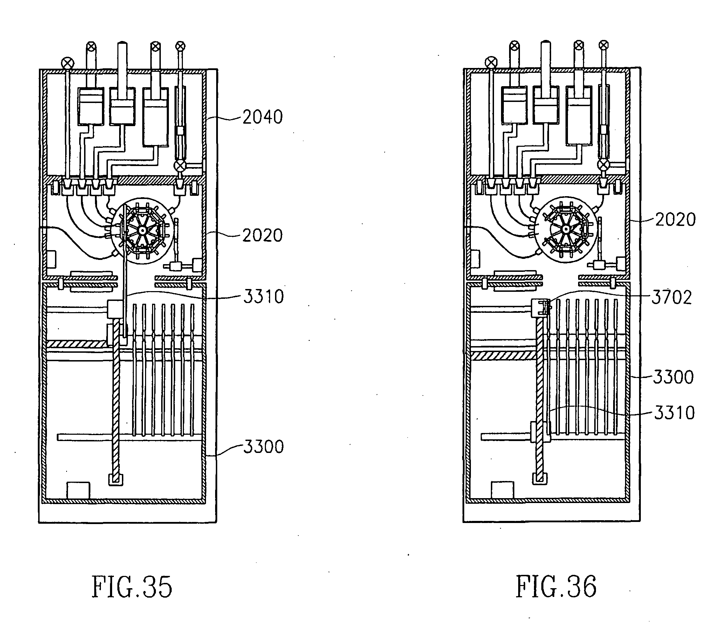

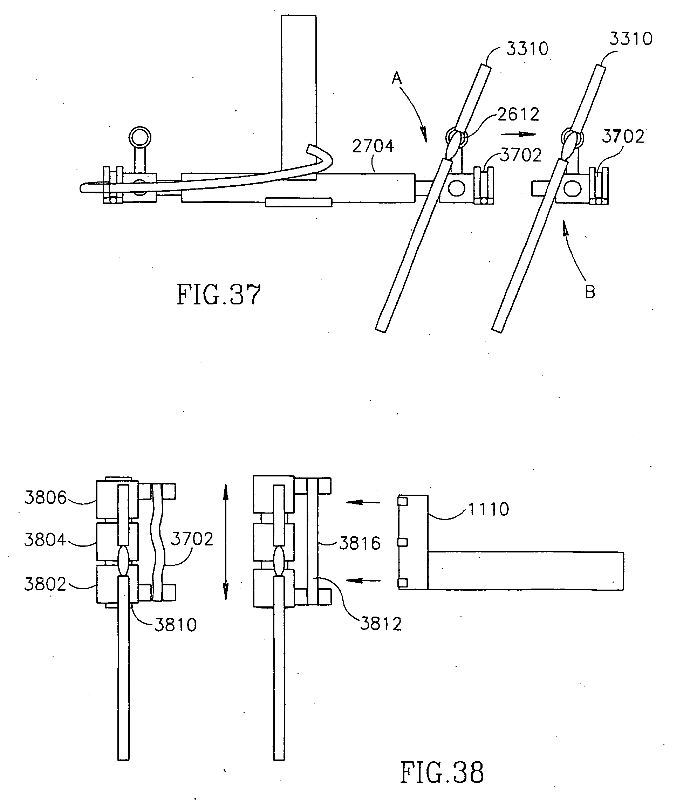

Fig. 35 shows one of pins 3310, extended into module 2020 and engaging one micro-organ segment mount 2610 . Fig. 37 (at A) shows pin 3310 engaging eye 2612 on the top of segment mount 2610. A slight rotation of micro-organ holder 2704 or a lateral motion of pin 3310 (by the x-y stage) causes the segment mount (together with an micro-organ/TMO segment 3702) to detach from holder 2704, so that the micro-organ segment 3702 is held by pin 3310 (at B). Eyelet 2612 is shown as a circular structure, but could also be tubular or other to assist in grasping the leading portion of pin 3110.

Pin 3310, together with segment 3702 can then be withdrawn into module 3300, as shown in Fig. 36 by means of the x-y stage. This process can be repeated for any desired number of segments, as needed for implantation, by loading another pin onto the x-y stage and grasping another micro-organ segment from module 2020. The ports are closed and module 2020, together with module 2040 can then be returned to a docking station on the right side of Fig. 29 for continued maintenance of any unloaded micro-organs/TMOs.

The entire TMO transfer module 3300 is disconnected from the assembly, and transported to a treatment center, optionally with provided control of the temperature, humidity, gases, and other environmental parameters. Module 3300 is optionally capable of manual operation to remove desired pins 3310.

When a micro-organ/TMO is to be implanted the pin is removed from module 3300 and transferred to a tool 1110 for implantation (Fig. 38), as described with respect to Fig. 11. Of course, other implantation methods, as described in the art and as described herein can also be used.

Micro-organ/TMO administration is typically performed in a clinically clean room such as a outpatient clinic or operating room. Module 3300 is typically to be used manually in the treatment room in the presence of the subject.

Each pin 3310 removed may undergo a straightening procedure, for example, as shown in Fig. 38. If the micro-organ/TMO segment 3702 has undergone relaxation during its processing, resulting in a segment that is not sufficiently straight for then next steps, as shown in the left figure, straightening can be achieved as follows.

Note that each segment mount is comprised of three sections, shown as 3802, 3804, and 3806, on a common ratchet rod 3810. By pulling section 3802 and/or 3806 away from central section 3804, tension can be applied to the TMO segment, resulting in straightened segment 3812.

The straight micro-organ/TMO segment can now be removed with reliable orientation from the segment mount. This is typically done using a vacuum pickup tool 1110, intended for use in conjunction with the method described with respect to Fig. 11. Tool 1110 is brought into close contact with straight segment 3812 while connected to vacuum source, so that it can be held against the stratum corneum 3816 side of the straight segment 3812 and hold the micro-organ/TMO by means of its vacuum holes.

This process is repeated until the requisite number of micro-organs/TMOs has been administered to the subject.

Typically, the bio-reactor is maintained at near body temperature (for example, 36-38°C, at high humidity preferably 95 %, and in a CO₂ enriched atmosphere (3-10% CO₂, 90-97% air). Optionally, the micro-organ is conditioned with antibiotics, anti-fungal and/or other agents. Chemicals or reagents required for accurate measure of protein secretion may be kept in refrigerated storage while awaiting use.

A control center for inputting commands and receiving data is also optionally provided. Controller 2940 is provided with software to make the process automatic or quasi-automatic and to provide data to the display.

It will thus be clear, the present invention has been described using non-limiting detailed descriptions of exemplary embodiments thereof that are provided by way of example and that are not intended to limit the scope of the invention. In particular, the systems described have been shown in great detail. It will be evident to persons of skill in the art that many of the operations described can be performed by other means, and that many of the acts described and features shown are not absolutely necessary.

For example, only a limited number of genetic changes have been shown. However, based on the methodology described herein in which live tissue is replanted in the body of the patient, and the viability of that tissue in the body after implantation, it is clear that virtually any genetic change in the tissue, induced by virtually any known method will result in secretions of target proteins or other therapeutic agents in the patient.

Variations of embodiments of the invention, including combinations of features from the various embodiments will occur to persons of the art. The scope of the invention is thus limited only by the scope of the claims. Furthermore, to avoid any question regarding the scope of the claims, where the terms "comprise" "include," or "have" and their conjugates, are used in the claims, they mean "including but not necessarily limited to".

## Claims

1. A micro-organ processing system, comprising:
a plurality of operational modules, each said module performing all or part of a process of producing one or more micro-organs from a viable tissue explant, wherein said micro-organ is cut from the explant and is a viable tissue section having intact micro-architecture;
wherein one of said modules is a harvester module comprising a means for harvesting said tissue explant from a tissue, and
wherein at least one of said modules comprises an inlet for nutrients and an outlet for waste in order that said explant and/or said one or more micro-organs can be maintained; and
means for transferring the tissue explant or said one or more micro-organs from one module to a next module in micro-organ processing system, wherein said transferring is via ports in the modules and said means is carried out under sterile conditions.

2. A micro-organ processing system according to claim 1, wherein said at least one module comprising an inlet for nutrients and an outlet for waste is fitted with an inlet for supplying a transduction agent, such that the micro-organ can be genetically altered therein..

3. A micro-organ processing system according to claim 1, wherein said at least one module comprising an inlet for nutrients and an outlet for waste is fitted with a sampling outlet for sampling the surrounding fluid therein.

4. A micro-organ processing system according to claim 1, wherein said plurality of modules is supplied with matching ports and connecting mechanisms such that material can be transported between said modules without exposure to an outside environment.

5. A micro-organ processing system according to claim 1 wherein said plurality of modules carry out the process of producing one or more micro-organs under sterile conditions starting from the introduction of the tissue explant.

6. A micro-organ processing station for the maintenance and optional genetic alteration of micro-organs, comprising:
the micro-organ processing system of claim 1;
at least one port for docking a module or a plurality of linked modules;
a fluidics control system operative to control the flow of fluids to and from at least one of the modules; and
a power control system operative to supply motive power to elements within at least one of the modules.

7. A micro-organ processing station according to claim 6, comprising a vacuum control system operative to supply a controlled vacuum to at least one of the modules, wherein said vacuum is for holding a tissue explant and/or said one or more micro-organs within at least one of the modules.

8. A micro-organ processing station according to claim 6, wherein the fluidics control system is operative to control the introduction of at least one material that causes the genetic alteration of said one or more micro-organs in one of the modules.

9. A micro-organ processing station according to claim 6 comprising a sampling mechanism for sampling fluids from at least one of said plurality of modules.

10. A micro-organ processing station according to claim 6, comprising an analyzer for analyzing the fluids, for one or more of the process parameters including glucose, lactate, dissolved oxygen, dissolved carbon dioxide, ammonia, glutamine, pH, contaminants or a secreted therapeutic agent.

11. A micro-organ processing station according to claim 10, wherein the analyzer analyzes the fluids for said therapeutic agent secreted by said one or more micro-organs.

12. A micro-organ processing station according to claim 11, comprising a controller for monitoring the amount of said therapeutic agent and indicating when the one or more micro-organs is suitable for implantation.

13. A micro-organ processing station according to claim 6, comprising a means for enhancing the genetic alteration of the one or more micro-organs.

14. A micro-organ processing station according to claim 13, wherein the means for enhancing comprises mechanical agitation, electroporation or electromagnetic field creation, or any combination thereof.

15. A micro-organ processing system according to claim 1, wherein one of said plurality of modules is a micro-organ module comprising a means for cutting said tissue explant into said one or more micro-organs.

## Patentansprüche

1. Mikroorgan-Verarbeitungssystem, umfassend:
eine Vielzahl von operativen Modulen, wobei jedes Modul einen Prozess des Herstellens eines oder mehrerer Mikroorgane aus einem lebensfähigen Gewebeexplantat ganz oder teilweise durchführt, wobei das Mikroorgan von dem Explantat abgeschnitten wird und ein lebensfähiger Gewebeabschnitt mit intakter Mikroarchitektur ist;
wobei eines der Module ein Erntemodul ist, das ein Mittel zum Ernten des Gewebeexplantats aus einem Gewebe umfasst und
wobei mindestens eines der Module einen Einlass für Nährstoffe und einen Auslass für Abfallstoffe umfasst, so dass das Explantat und/oder das eine oder die mehreren Mikroorgane erhalten werden können; und
Mittel zum Übertragen des Gewebeexplantats oder des einen oder der mehreren Mikroorgane von einem Modul auf ein nächstes Modul in einem Mikroorgan-Verarbeitungssystem, wobei das Übertragen über Anschlüsse in den Modulen verläuft und das Mittel unter sterilen Bedingungen durchgeführt wird.

2. Mikroorgan-Verarbeitungssystem nach Anspruch 1, wobei das mindestens eine Modul, das einen Einlass für Nährstoffe und einen Auslass für Abfallstoffe umfasst, mit einem Einlass zur Zuführung eines Transduktionsmittel ausgestattet ist, so dass das Mikroorgan darin genetisch verändert werden kann.

3. Mikroorgan-Verarbeitungssystem nach Anspruch 1, wobei das mindestens eine Modul, das einen Einlass für Nährstoffe und einen Auslass für Abfallstoffe umfasst, mit einem Probenauslass zur Probenahme des sich darin befindlichen Fluids ausgestattet ist.

4. Mikroorgan-Verarbeitungssystem nach Anspruch 1, wobei die Vielzahl von Modulen mit passenden Anschlüssen und Verbindungsmechanismen versorgt ist, so dass Material zwischen den Modulen transportiert werden kann, ohne dabei der Außenwelt ausgesetzt zu sein.

5. Mikroorgan-Verarbeitungssystem nach Anspruch 1, wobei die Vielzahl von Modulen den Prozess des Herstellens eines oder mehrerer Mikroorgane unter sterilen Bedingungen beginnend mit der Einführung des Gewebeexplantats durchführt.

6. Mikroorgan-Verarbeitungsstation zur Erhaltung und wahlweisen genetischen Veränderung von Mikroorganen, umfassend:
das Mikroorgan-Verarbeitungssystem nach Anspruch 1;
mindestens einen Anschluss zum Koppeln eines Moduls oder einer Vielzahl verbundener Module;
ein Fluidik-Steuersystem, das betreibbar ist, um den Fluss von Fluiden zu und von mindestens einem der Module zu steuern; und
ein Leistungs-Steuersystem, das betreibbar ist, um Elementen innerhalb mindestens einem der Module, Antriebsleistung zu liefern.

7. Mikroorgan-Verarbeitungsstation nach Anspruch 6, umfassend ein Vakuum-Steuersystem, das betreibbar ist, um mindestens einem der Module ein kontrolliertes Vakuum zu liefern, wobei das Vakuum vorgesehen ist, ein Gewebeexplantat und/oder das eine oder die mehreren Mikroorgane innerhalb mindestens einem der Module zu halten.

8. Mikroorgan-Verarbeitungsstation nach Anspruch 6, wobei das Fluidik-Steuersystem betreibbar ist, um die Einführung von mindestens einem Material, das die genetische Veränderung des einen oder der mehreren Mikroorgane in einem der Module verursacht, zu steuern.

9. Mikroorgan-Verarbeitungsstation nach Anspruch 6, umfassend einen Probenentnahmemechanismus zum Entnehmen einer Fluidprobe aus mindestens einem der Vielzahl von Modulen.

10. Mikroorgan-Verarbeitungsstation nach Anspruch 6, umfassend einen Analysator zum Analysieren der Fluide hinsichtlich eines oder mehrerer der Prozessparameter, einschließlich Glucose, Lactat, gelöstem Sauerstoff, gelöstem Kohlendioxid, Ammoniak, Glutamin, pH, Verunreinigungen oder eines sekretierten therapeutischen Mittels.

11. Mikroorgan-Verarbeitungsstation nach Anspruch 10, wobei der Analysator die Fluide hinsichtlich des therapeutischen Mittels analysiert, das von dem einen oder den mehreren Mikroorganen sekretiert wird.

12. Mikroorgan-Verarbeitungsstation nach Anspruch 11, umfassend eine Steuerung zum Überwachen der Menge des therapeutischen Mittels und Anzeigen, wenn das eine oder die mehreren Mikroorgane zur Implantation geeignet sind.

13. Mikroorgan-Verarbeitungsstation nach Anspruch 6, umfassend ein Mittel zur Verstärkung der genetischen Veränderung des einen oder der mehreren Mikroorgane.

14. Mikroorgan-Verarbeitungsstation nach Anspruch 13, wobei das Mittel zum Verstärken mechanisches Schütteln, Elektroporation oder Erzeugung eines elektromagnetischen Felds umfasst.

15. Mikroorgan-Verarbeitungsstation nach Anspruch 1, wobei eines der Vielzahl von Modulen ein Mikroorganmodul ist, das ein Mittel zum Schneiden des Gewebeexplantats in das eine oder die mehreren Mikroorgane umfasst.

## Revendications

1. Système de traitement de micro-organe, comprenant :
une pluralité de modules opérationnels, chaque dit module exécutant la totalité ou une partie d'un procédé de production d'un ou plusieurs micro-organes à partir d'un explant de tissu viable, dans lequel ledit micro-organe est coupé de l'explant et est une section de tissu viable ayant une micro-architecture intacte ;
dans lequel un desdits modules est un module récolteur comprenant un moyen pour récolter ledit explant de tissu d'un tissu, et
dans lequel au moins l'un desdits modules comprend une entrée pour des nutriments et une sortie pour les déchets afin que ledit explant et/ou ledit ou lesdits micro-organes puissent être maintenus ; et
un moyen de transfert de l'explant de tissu ou dudit un ou plusieurs micro-organes d'un module à un module suivant dans un système de traitement de micro-organe, dans lequel ledit transfert s'effectue par l'intermédiaire d'orifices dans les modules et ledit moyen est exécuté dans des conditions stériles.

2. Système de traitement de micro-organe selon la revendication 1, dans lequel ledit au moins un module comprenant une entrée pour des nutriments et une sortie pour les déchets est équipé d'une entrée pour alimenter un agent de transduction, de telle sorte que le micro-organe peut y être génétiquement modifié.

3. Système de traitement de micro-organe selon la revendication 1, dans lequel ledit au moins un module comprenant une entrée pour des nutriments et une sortie pour les déchets est équipé d'une sortie d'échantillonnage pour échantillonner le fluide environnant qui s'y trouve.

4. Système de traitement de micro-organe selon la revendication 1, dans lequel ladite pluralité de modules est munie d'orifices d'adaptation et mécanismes de raccordement de telle sorte que du matériau peut être transporté entre lesdits modules sans exposition à un environnement extérieur.

5. Système de traitement de micro-organe selon la revendication 1, dans lequel ladite pluralité de modules exécute le procédé de production d'un ou plusieurs micro-organes dans des conditions stériles en partant de l'introduction de l'explant de tissu.

6. Station de traitement de micro-organe pour le maintien et la modification génétique facultative de micro-organes, comprenant :
le système de traitement de micro-organe selon la revendication 1 ;
au moins un orifice pour accueillir un module ou une pluralité de modules liés ;
un système de commande fluidique opérationnel pour commander l'écoulement de fluides vers et à partir d'au moins un des modules ; et
un système de contrôle de puissance opérationnel pour fournir une puissance motrice aux éléments au sein d'au moins un des modules.

7. Station de traitement de micro-organe selon la revendication 6, comprenant un système de commande de vide opérationnel pour fournir un vide commandé à au moins un des modules, dans laquelle ledit vide sert à tenir un explant de tissu et/ou ledit ou lesdits micro-organes au sein d'au moins un des modules.

8. Station de traitement de micro-organe selon la revendication 6, dans laquelle le système de commande fluidique est opérationnel pour commander l'introduction d'au moins un matériau qui provoque la modification génétique desdits un ou plusieurs micro-organes dans un des modules.

9. Station de traitement de micro-organe selon la revendication 6, comprenant un mécanisme d'échantillonnage pour échantillonner des fluides provenant d'au moins l'un parmi ladite pluralité de modules.

10. Station de traitement de micro-organe selon la revendication 6, comprenant un analyseur pour analyser les fluides, pour détecter un ou plusieurs des paramètres de procédé incluant le glucose, le lactate, l'oxygène dissous, le dioxyde de carbone dissous, l'ammoniac, la glutamine, le pH, les contaminants ou un agent thérapeutique sécrété.

11. Station de traitement de micro-organe selon la revendication 10, dans laquelle l'analyseur analyse les fluides pour détecter ledit agent thérapeutique sécrété par ledit un ou lesdits plusieurs micro-organes.

12. Station de traitement de micro-organe selon la revendication 11, comprenant un contrôleur pour surveiller la quantité dudit agent thérapeutique et indiquer lorsque le ou les micro-organes sont appropriés pour implantation.

13. Station de traitement de micro-organe selon la revendication 6, comprenant un moyen pour renforcer la modification génétique du ou des micro-organes.

14. Station de traitement de micro-organe selon la revendication 13, dans laquelle le moyen pour renforcer comprend une agitation mécanique, une électroporation ou une création de champ électromagnétique, ou n'importe quelle combinaison de celles-ci.

15. Système de traitement de micro-organe selon la revendication 1, dans lequel un parmi ladite pluralité de modules est un module de micro-organe comprenant un moyen pour couper ledit explant de tissu en ledit un ou lesdits plusieurs micro-organes.
